# EUROPEAN PATENT SPECIFICATION

(11) **EP 4 129 206 B1**
(45) Date of publication and mention of the grant of the patent: **25.09.2024**
(21) Application number: 21780517.5
(22) Date of filing: 30.03.2021
(51) Int. Cl.: A61B 17/072, A61B 50/30, A61B 17/00, A61B 17/32

(54) **SURGICAL KIT**
CHIRURGISCHES KIT
KIT CHIRURGICAL

(30) Priority: 03.04.2020 CN 202010261072
(43) Date of publication of application: 08.02.2023
(73) Proprietor: Fulbright Medical Inc., Wuxi, Jiangsu 214437 (CN)
(72) Inventor: SUN, Baofeng, Wuxi, Jiangsu 214437 (CN); FU, Jianxin, Wuxi, Jiangsu 214437 (CN); ZHOU, Xiaopeng, Wuxi, Jiangsu 214437 (CN)
(74) Representative: Zacco GmbH
(86) International application number: PCT/CN2021/083993
(87) International publication number: WO 2021/197329

(56) References cited:
- EP-A1- 2 236 105
- EP-A1- 3 403 595
- EP-A1- 3 449 851
- EP-A2- 3 597 119
- WO-A1-2017/048776
- CN-A- 101 317 779
- CN-A- 101 856 256
- CN-A- 102 048 566
- CN-A- 110 251 178
- CN-U- 212 346 615
- CN-U- 212 346 616
- CN-U- 212 490 027
- US-A- 5 842 567
- US-A1- 2013 299 371
- US-A1- 2019 008 515
- US-A1- 2019 365 383

## Description

### Technical Field

The present disclosure relates to the technical field of medical apparatus and instruments, and in particular, to an assembly for a surgical instrument. The assembly includes a separation member used for separating target tissue and a packaging box for storing the separation member.

The present invention relates to a surgical kit. The surgical kit includes a surgical instrument and the above assembly.

### Background

A stapler suitable for surgery is a surgical instrument that can excise redundant tissue while suturing a wound of a patient, and is widely applied to resection and anastomosis of tissue in minimally invasive surgery such as abdominal surgery, gynecology departments, pediatric departments and thoracic surgery. The stapler enters a human body by using a cannula of a puncture outfit that accurately positions a surgical site, and then makes a longitudinal incision in the tissue and applies a suturing staple on an opposite side of the incision, so as to dissect and anastomose the tissue, which is similar to a book sewer. The stapler includes an end effector. The end effector includes a staple cartridge base and a staple abutting seat. The staple cartridge base is used to receive a staple cartridge. The stapler further includes a cutting component. The cutting component is operationally supported relative to the staple cartridge base. Once a doctor determines that the end effector is appropriately grasp the tissue and in a closed position, the stapler can be started to cut and suture the tissue.

During surgery, the doctor sometimes needs to cut and suture specific target tissue, but the target tissue is attached to other tissue. For example, the doctor sometimes needs to cut and suture a blood vessel, but the blood vessel is usually attached to other tissue. In order to cut and suture the blood vessel, the doctor needs to strip the blood vessel from other tissue, then makes the blood vessel be between the staple abutting seat and the staple cartridge base, and finally uses the stapler to cut and suture the blood vessel.

When the doctor operates a conventional surgical instrument, and if the doctor needs to cut and suture the specific target tissue, the doctor usually needs to repeatedly adjust an angle of the surgical instrument to cause a far end of the end effector to align the target tissue, applies force to the surgical instrument in a direction toward the far end, to cause the far end of the end effector to separate the target tissue and then to cause the target tissue to be between the staple abutting seat and the staple cartridge base, so as to cut and suture the target tissue.

However, the design of the far end of the conventional end effector is not suitable for the stripping of the target tissue, and it is laborious for the doctor to use the end effector of the surgical instrument to separate the target tissue.

EP 2 236 105 A1 discloses a package for a surgical blade tip is provided. The package cooperates with the surgical blade tip to properly retain it and facilitate mounting onto a surgical blade handle. In one aspect, the invention includes a package having a body with a side wall and at least two detents extending inwardly from the side wall. In addition, a surgical blade tip is provided having an adaptor with a blade extending there from the adaptor includes at least two recesses formed therein. The surgical blade tip is disposed in the package with the detents being at least partially inserted into the recesses. Also, the detents are configured to apply a retentive force to the adaptor to retain the adaptor in a first position and configured to deflect with a predetermined amount of torque being applied to the surgical blade tip so as to permit the adaptor to rotate from the first position to a second position where the detents are not inserted into the recesses. Advantageously, with the subject invention, a package may be provided which maintains a blade of a surgical blade tip in spaced relationship so as to minimize contamination thereof. In addition, the inter engagement of the detents and the recesses allows for proper retention of the surgical blade tip during mounting onto a surgical blade handle.

EP 3 449 851 A1 discloses a blade loader is provided herein. The blade loader includes a housing defining a cavity and an insertion opening at one end portion of the cavity. The cavity is configured to accept a blade therein. The housing includes first and second sections. First and second sidewalls extend from the respective first and second sections. The first sidewall is disposed outside the second sidewall. A support extends from the housing and into the cavity configured to interact with an aperture defined by the blade. A rib structure extends into the cavity and defines a channel and a press portion. The press portion is configured to contact a portion of the blade.

### Summary

The invention is set out in the appended set of claims.

In view of the deficiency in the prior art, the present invention is intended to provide an assembly for a surgical instrument. The assembly includes a separation member used for separating target tissue and a packaging box for storing the separation member.

The present invention is achieved by the following technical solutions. The assembly for a surgical instrument includes a separation member and a packaging box. The separation member and a surgical instrument switch between a first state and a second state by means of a clipping mechanism. In the first state, the separation member is connected to the surgical instrument. In the second state, the separation member is detached from the surgical instrument. The packaging box includes an accommodation portion used for accommodating the separation member, and further includes an operating member. The operating member drives the clipping mechanism to switch the separation member from the first state to the second state.

In some embodiments, the operating member includes an operating portion and a driving portion. The operating portion is pressed by an operator. The driving portion drives the clipping mechanism to switch the separation member from the first state to the second state.

In some embodiments, the operating portion performs first movement to drive the driving portion to perform second movement, so as to switch the separation member from the first state to the second state.

In some embodiments, the first movement includes rotation; and the second movement includes rotation.

In some embodiments, the packaging box includes a housing. The accommodation portion is disposed to the housing. The housing further includes a first sidewall and a second sidewall surrounding the accommodation portion. There is a gap between the first sidewall and the second sidewall. The first sidewall forms the operating portion.

In some embodiments, the operating portion includes a free end and a connection end connected to the second sidewall. The free end rotates around a junction of the connection end and the second sidewall, to drive the driving portion to move.

In some embodiments, a rotation axis of the free end is perpendicular to a central symmetry plane in a longitudinal direction of the packaging box.

In some embodiments, at least one portion of the operating portion is made of an elastic material, and the at least one portion of includes the connection end.

In some embodiments, an extending direction of the operating portion is perpendicular to an extending direction of the driving portion.

In some embodiments, the packaging box includes a housing. The housing is provided with an opening hole. The opening hole is disposed opposite to the driving portion.

In some embodiments, the accommodation portion is provided with a guide surface. The guide surface is used to guide the separation member to be inserted into the accommodation portion along the guide surface.

In some embodiments, the accommodation portion is provided with an abutment surface extending lengthwise. The abutment surface is used to limit the separation member.

In some embodiments, the accommodation portion is further provided with a guide surface. The guide surface is used to guide the separation member to move into the accommodation portion along the guide surface. The abutment surface and the guide surface intersect with each other and are disposed at an angle.

In some embodiments, the accommodation portion has an open end and a closed end. The open end is used for inserting the separation member into the accommodation portion. The closed end is used to limit the separation member.

In some embodiments, a protrusion is disposed on a surface of the accommodation portion.

In some embodiments, the clipping mechanism includes a clipping portion disposed to the separation member and a clipping port disposed to the surgical instrument. In the first state, the clipping portion is in elastically-mated connection with the clipping port. The operating member drives the clipping portion to detach from the clipping port, so that the separation member switches from the first state to the second state.

In some embodiments, the packaging box is made of a transparent material.

Compared with the prior art, the beneficial effects of the present invention are that, the assembly of the present invention includes the separation member and the packaging box. The separation member is used for stripping the target tissue, to make the operation of a doctor easier and more convenient. The packaging box can not only provide packaging for the separation member, but also serve as a disassembling tool of the separation member relative to the surgical instrument, of which design is very artful.

The present invention further provides to a surgical kit. The surgical kit includes a surgical instrument and the above assembly.

The present invention is achieved by the following technical solutions. The surgical kit includes the surgical instrument and the assembly described in any one of the above. A separation member is mounted to the surgical instrument to strip tissue.

In some embodiments, the separation member is detachably mounted to the surgical instrument by using a mating mechanism. The mating mechanism includes an insertion portion disposed to the separation member and an insertion channel disposed to the surgical instrument. The mating mechanism further includes a clipping portion disposed to the separation member and a clipping port disposed to the surgical instrument. When the clipping portion and the insertion portion are inserted into the clipping portion to be in clipped connection with the clipping port, the separation member is fixed to the surgical instrument.

In some embodiments, a sidewall of the insertion channel is provided with a channel groove extending from a far end to a near end. An operating member includes an operating portion and a driving portion. The driving portion drives the clipping portion to detach from the surgical instrument. A width of the driving portion is less than a width of the channel groove.

Compared with the prior art, the beneficial effects of the present invention are that, the surgical kit includes the assembly and the surgical instrument, which can meet requirements of the doctor for separating the target tissue and cutting and suturing the target tissue; and the surgical kit is convenient in operation.

### Brief Description of the Drawings

Fig. 1 is a front view of a surgical instrument according to the present invention.
Fig. 2 is a perspective matching view of a staple abutting seat of the surgical instrument shown in Fig. 1 and a separation member, showing a front side and partial back side, respectively.
Fig. 3 and Fig. 4 are three-dimensional views of the separation member shown in Fig. 2.
Fig. 5 is a three-dimensional exploded view of the separation member shown in Fig. 4.
Fig. 6 is a partial three-dimensional view of a staple abutting seat of the surgical instrument shown in Fig. 1.
Fig. 7 and Fig. 8 are three-dimensional views of a combination formed by a staple abutting seat, a separation member and a packaging box.
Fig. 9 and Fig. 10 are three-dimensional exploded views of the combination shown in Fig. 8.
Fig. 11 is a three-dimensional view of a packaging box shown in Fig. 10.
Fig. 12 and Fig. 13 are cross-sectional views in a longitudinal direction in Fig. 7; and state changes of Fig. 12 to Fig. 13 show a state changing process of using a packaging box to detach a separation member.
Fig. 14 is an enlarged view of a part A in Fig. 12.
Fig. 15 is an enlarged view of a part B in Fig. 12.
Fig. 16 is a three-dimensional view of a separation member according to a second implementation of the present invention. Fig. 17 is a three-dimensional exploded view of the separation member shown in Fig. 16.
Fig. 18 is a three-dimensional exploded view of a separation member according to a third implementation of the present invention.
Fig. 19 is a three-dimensional view of a separation member according to a fourth implementation of the present invention.
Fig. 20 is a three-dimensional exploded view of the separation member shown in Fig. 19.
Fig. 21 is a three-dimensional view of a separation member mounted to a staple abutting seat according to a fifth implementation of the present invention.
Fig. 22 is a three-dimensional view of the separation member shown in Fig. 21.
Fig. 23 is a three-dimensional view of a separation member mounted to a staple abutting seat according to a sixth implementation of the present invention.
Fig. 24 and Fig. 25 are three-dimensional exploded views of Fig. 23.
Fig. 26 is a three-dimensional view of the separation member shown in Fig. 23.
Fig. 27 is a three-dimensional view of a separation member mounted to a staple abutting seat according to a seventh implementation of the present invention.
Fig. 28 is a three-dimensional view of the separation member shown in Fig. 27.
Fig. 29 is a three-dimensional exploded view of the separation member shown in Fig. 28.

### Detailed Description of the Embodiments

To make the objectives, technical solutions and advantages of the present invention clearer, the present invention is further described in detail with reference to the drawings and embodiments. It should be understood that the specific examples described here are merely used to explain the present invention, and are not used to limit the present invention. Based on the embodiments in the present invention, all other embodiments obtained by those of ordinary skilled in the art without creative work shall fall within the protection scope of the present invention.

The terms "near", "rear", "far" and "front" are used herein with respect to a clinician manipulating a handle assembly of a surgical instrument. The terms "near" and "rear" refer to portions close to the clinician; and the terms "far" and "front" refer to portions away from the clinician. For example, the handle assembly is on a near side and a rear side, and an end effector is on a far side and a front side. For another example, a near side end of a certain part represents an end relatively close to the handle assembly, and a far side end represents an end relatively close to the end effector. In the present invention, a relative positional relationship between the parts of the separation member, for example, being relatively located on a "near" end or a "far" end, is based on the state in which the separation member is mounted to the surgical instrument. The terms "upper" and "lower" are based on relative positions of a staple abutting seat and a staple cartridge base of the end effector. Specifically, the staple abutting seat is at the "upper" portion, and the staple cartridge base is at the "lower" portion.

The "longitudinal direction" is defined as a direction from far to near or a direction from near to far. The "longitudinal direction" of the separation member is defined according to the state in which the separation member is mounted to the surgical instrument. The "longitudinal direction" of the packaging box is defined according to the state in which the separation member is mounted to the packaging box, that is, the longitudinal direction of the packaging box is parallel to the longitudinal direction of the separation member. In addition, that a component extends lengthwise means that a length of the component is greater than a width of the component, for example, the entire component is long-strip-shaped. However, it is to be understood that, "lengthwise extending" does not limit the specific shape and setting position of the component.

A surface of the separation member close to a side of target tissue is defined as a "front surface", and a surface away from the side of the target tissue is defined as a "back surface". Specifically, when the separation member is operated, a concave surface of the working portion is close to the target tissue and can strip the target tissue from other tissues. The surface of the side where the concave surface is located is defined as the front surface, and the surface of the side where a convex surface is located is defined as the back surface.

It is to be understood that, the orientations "near", "far", "rear", "front", "upper", "lower", "front surface", "back surface" are defined for ease of description. However, the surgical instrument may be used in various directions and positions. Therefore, these terms expressing relative positional relationships are not restrictive and absolute. The "longitudinal direction" is also defined for ease of description. In the present invention, the above definitions shall comply with the above explicit stipulations and limitations if there are other explicit stipulations and definitions.

In the present invention, unless expressly specified and limited otherwise, the terms such as "connected to each other" should be understood in a broad sense. For example, "connected to each other" may be a fixed connection, a detachable connection, a movable connection, or an integrated; or may be a direct connection or an indirect connection by means of an intermediate medium; or may be an internal communication between two elements or an interaction relationship between two elements. Except that "connection" does not include integration, "connection" has the same meaning as "connected to each other". For those of ordinary skill in the art, specific meanings of the above terms in the present invention may be understood according to a specific condition.

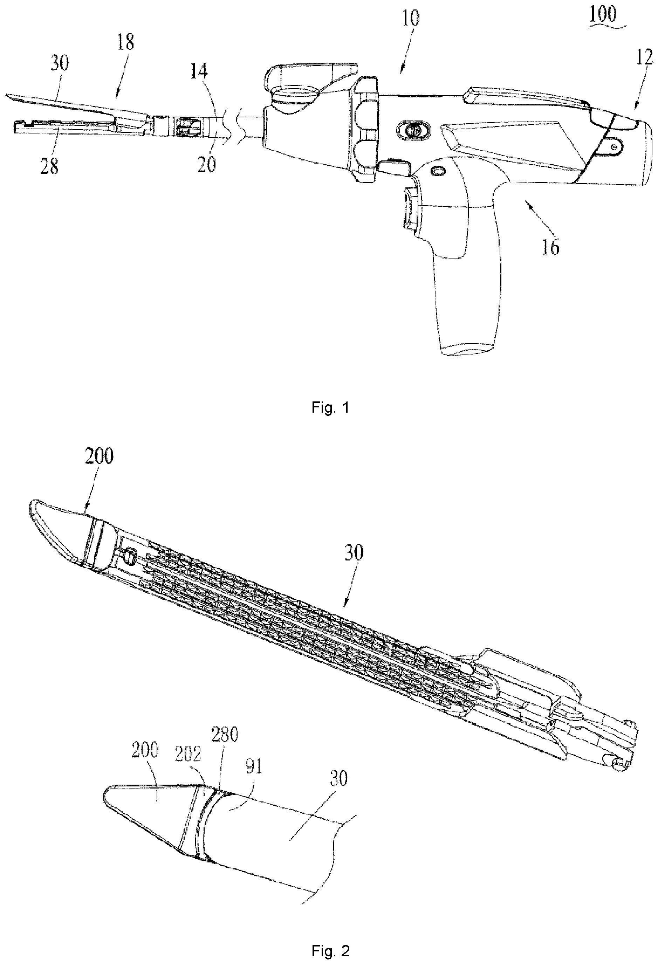

Fig. 1 shows a surgical instrument 100 in the present invention.

Referring to Fig. 1, the surgical instrument provided in the present invention is a stapler, preferably, an electric stapler. The surgical instrument 100 includes a host 10 and a battery pack 12 detachably mounted with the host 10. Those skilled in the art may think that, the surgical instrument may also be a manual stapler, and any solution that is the same as or similar to this implementation is covered within the protection scope of the present invention.

The host 10 includes a rod body assembly 14, a housing 16 disposed on one end of the rod body assembly 14, and an end effector 18 disposed on the other end of the rod body assembly 14. A cutting component (not shown) is disposed in the end effector 18.

The rod body assembly 14 includes a cannula 20 and a core shaft (not shown) accommodated in the cannula 20. A motor and a transmission mechanism are disposed in the housing 16. The motor drives the end effector 18 and the cutting component by using the transmission mechanism. The end effector 18 includes a staple cartridge base 28 and a staple abutting seat 30 rotatably connected to the staple cartridge base 28. The staple cartridge base 28 is used to operationally support a staple cartridge assembly (not shown) located therein. The staple abutting seat 30 may be selectively moved between an open position and a closed position, so as to clamp the tissue or loosen the tissue. The cutting component is disposed in the end effector 18 to cut the tissue. The staple cartridge assembly is used to suture the tissue.

The transmission mechanism includes an end effector driving apparatus. The end effector driving apparatus drives the staple abutting seat 30 of the end effector 18 to rotate relative to the staple cartridge base 28, so as to be selectively moved between the open position and the closed position. Generally, the end effector 18 driving apparatus includes a gear, a cam and the cannula 20. The gear drives the cannula 20 to do linear movement by means of the switching of the cam. By means of the linear movement of the cannula 20, the staple abutting seat 30 is driven to rotate by using a switching mechanism. A specific structure of the end effector driving apparatus is the same as or similar to that in the prior art, which is not described herein again.

The transmission mechanism further includes a cutting component driving apparatus. Generally, the cutting component driving apparatus includes a gear, a rack and a core shaft. The gear rotates to drive the rack, the core shaft and the cutting component to do linear movement, so that the cutting component may cut the tissue. A specific structure of the cutting component driving apparatus is the same as or similar to that in the prior art, which is not described herein again.

When the surgical instrument is used, the end effector driving apparatus first drives the cannula 20 to drive the staple abutting seat 30 to rotate to be mated with the staple cartridge base 28, so as to clamp the target tissue. Then, the motor starts and rotates forward; the cutting component driving apparatus drives the cutting component to advance by using the core shaft; and the cutting component moves from an initial position to a termination position in the end effector 18 of the surgical instrument, and synchronously pushes a staple-pushing block in the staple cartridge to move from the initial position to the termination position, so as to achieve an effect of suturing the cut tissue while redundant tissue is cut. Next, the motor rotates backward, and the cutting component driving apparatus drives the cutting component to retract to the initial position by using the core shaft. Then, the end effector driving apparatus drives the cannula 20 to drive the staple abutting seat 30 to open, so as to loosen the tissue. Finally, the end effector driving apparatus drives the cannula 20 to drive the staple abutting seat 30 to close. Definitely, before and after the surgical instrument is used, the staple abutting seat 30 is required to be closed relative to the staple cartridge base 28, so that the end effector 18 extends into a body of a patient or is removed from the body of the patient via the cannula of a puncture outfit, and details are not described again.

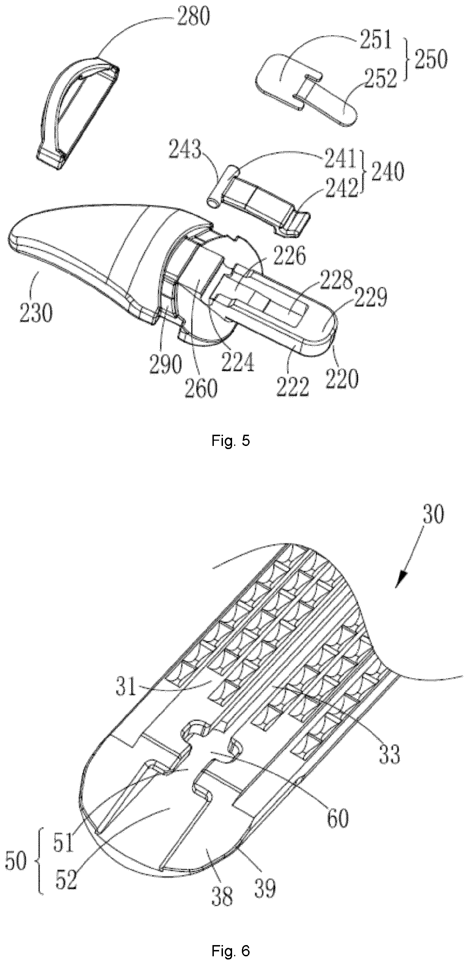

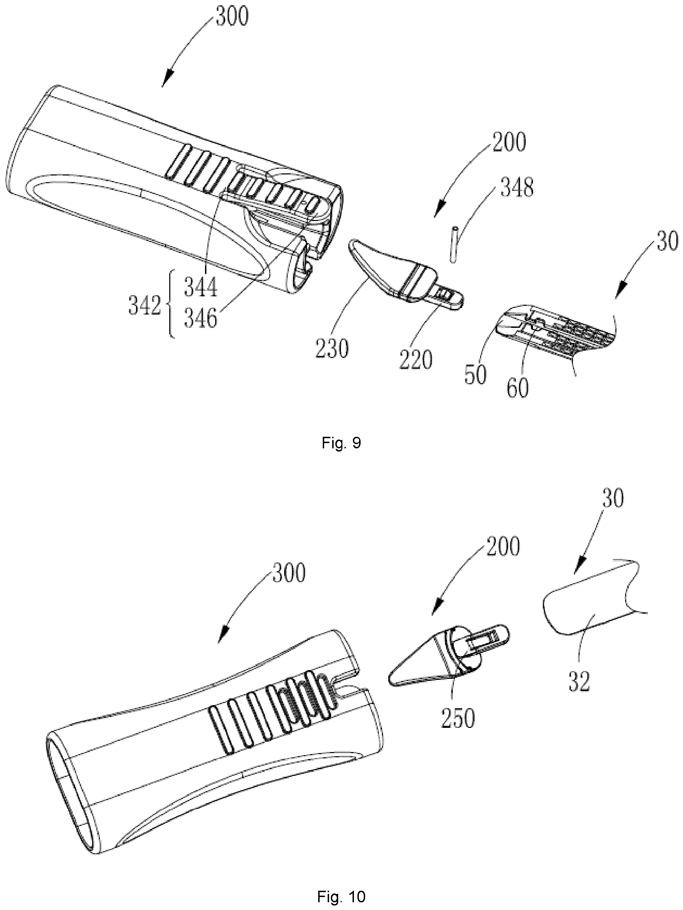

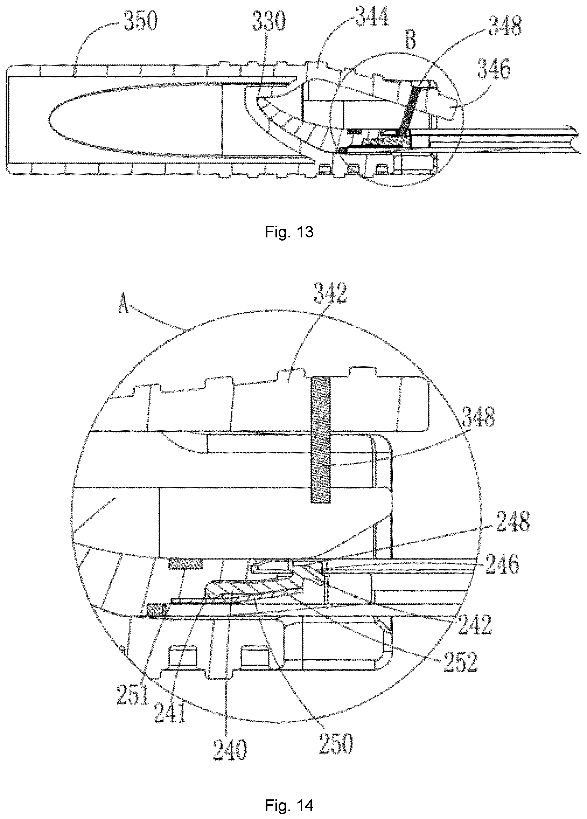

Fig. 2 to Fig. 15 show the separation member in a first implementation of the present invention. The separation member is used in cooperation with the surgical instrument 100.

Referring to Fig. 1 and Fig. 2, this implementation further provides the separation member 200 cooperatively used with the surgical instrument 100. The separation member 200 is used to strip the tissue. Specifically, the separation member 200 is used to strip the target tissue from other tissue, so that the surgical instrument 100 may conveniently cut and suture the target tissue. For example, a doctor first uses the separation member 200 to strip the blood vessel from other tissue, and then uses the surgical instrument 100 to cut and suture the stripped blood vessel.

The separation member 200 is detachably connected to the surgical instrument 100. That is to say, the separation member 200 switches between a first state and a second state. In the first state, the separation member 200 is connected to the surgical instrument 100; and in the second state, the separation member 200 is detached from the surgical instrument 100.

Therefore, when the separation member 200 is required to be used, the doctor connects the separation member 200 and the surgical instrument 100. The doctor first uses the separation member 200 to strip the target tissue from other tissue, and then moves the surgical instrument 100 forward, to cause the target tissue to be between the staple cartridge base 28 and the staple abutting seat 30, so as to operate the surgical instrument 100 to cut and suture the target tissue. When the separation member 200 is not required to be used, the doctor switches the separation member 200 from the first state to the second state, so that the separation member 200 is detached from the surgical instrument 100. In this way, the separation member 200 can be prevented from occupying surgical space to affect the convenience of the surgical instrument 100 for cutting and suturing other tissue.

Specifically, the separation member 200 is detachably connected to the end effector 18 of the surgical instrument 100. The end effector 18 is a working component in the surgical instrument 100 that is in direct contact with the tissue and cuts and sutures the tissue. That the separation member 200 is detachably connected to the end effector 18 conforms to the operation habits of the doctor and facilitates the operation of the doctor.

More specifically, the separation member 200 is detachably connected to the staple abutting seat 30 of the end effector 18. Since the staple abutting seat 30 of the conventional surgical instrument 100 is not equipped with the staple cartridge and other components, the separation member 200 is detachably connected to the staple abutting seat 30, so that the design is more rational by rationally using the staple abutting seat 30 without affecting the mounting and dismounting of other components (such as the staple cartridge). More specifically, the separation member 200 is detachably connected to a far end of the staple abutting seat 30, which is more conforms to the operation habits of the doctor and facilitates the operation of the doctor.

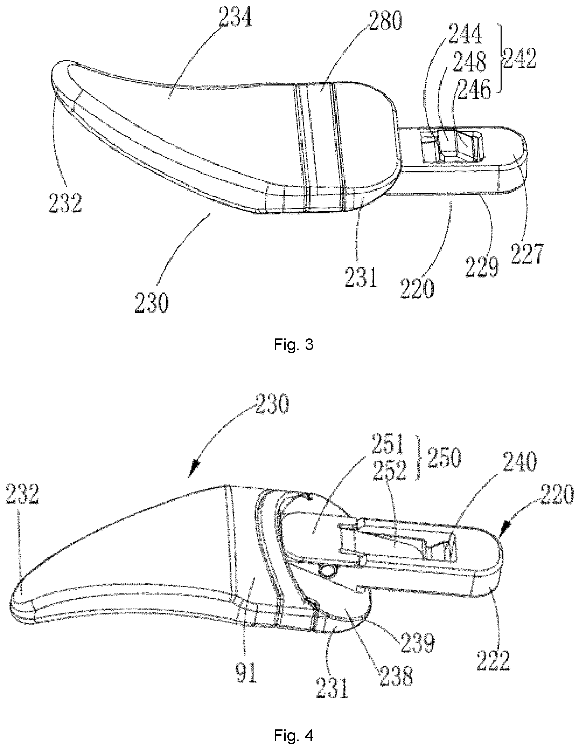

Referring to Fig. 3 to Fig. 5, the separation member 200 includes a mounting portion 220 and a working portion 230. The mounting portion 220 is detachably connected to the surgical instrument 100, and specifically, is detachably connected to the far end of the staple abutting seat 30 of the end effector 18. The working portion 230 is used to strip the tissue.

The mounting portion 220 is detachably connected to the surgical instrument 100 by using the mating mechanism. The mating mechanism includes an insertion mechanism and a clipping mechanism. The insertion mechanism includes an insertion portion 222 disposed to the separation member 200 and an insertion channel 50 disposed on the surgical instrument 100. The clipping mechanism includes a clipping portion 240 disposed on the separation member 200 and a clipping port 60 disposed on the surgical instrument 100. The insertion channel 50 communicates with the clipping port 60. Specifically, the clipping port 60 communicates with a near end of the insertion channel 50 and penetrates a sidewall of the insertion channel. When the separation member 200 is mounted, the insertion portion 222 and the clipping portion 240 are inserted into the surgical instrument 100 along the insertion channel 50. After a certain stroke is inserted, the clipping portion 240 is clipped in the clipping port 60, so that the separation member 200 is fixed on the surgical instrument 100. Therefore, the mounting portion 220 of the separation member 200 is detachably connected to the surgical instrument 100 through insertion and clipping, thereby achieving reliable mounting and convenient dismounting.

The mounting portion 220 includes the insertion portion 222 and the clipping portion 240 that are connected to each other. The clipping portion 240 is in elastically-mated connection with the surgical instrument 100. That is to say, the clipping portion 240 is connected to the surgical instrument 100 under the action of elastic force, and is detached from the surgical instrument 100 by overcoming the action of elastic force, so that a reliable connection and convenient dismounting can be realized. A source of the elastic force may be the elasticity of the clipping portion 240 itself. Alternatively, other elastomer may abut against the clipping portion 240, and provide the elastic force for the clipping portion 240.

Specifically, the clipping portion 240 is in elastically-mated connection with the clipping port 60. That is to say, the clipping portion 240 is clipped in the clipping port 60 under the action of elastic force, so that the separation member 200 is fixed to the surgical instrument 100. By overcoming the action of elastic force, the clipping portion 240 may be detached from the clipping port 60, so that the separation member 200 is detached from the surgical instrument 100.

The clipping portion 240 has a first end 241 and a second end 242. The first end 241 is connected to the insertion portion 222, and the second end 242 is in elastically-mated connection with the surgical instrument 100. Specifically, the second end 242 rotates around the first end 241, to cause the separation member 200 to be fixed to the surgical instrument 100 or to be detached from the surgical instrument, so as to achieve a detachable connection. The second end 242 rotates around the first end 241, that is, a rotation axis is located at a position of the first end 241. In this implementation, the rotation axis of the second end 242 is perpendicular to a central symmetry plane in a longitudinal direction of the separation member 200. In this implementation, the first end 241 is located on the far end relative to the second end 242. Those skilled in the art may think that, the first end 241 is located on the near end relative to the second end 242.

Specifically, the first end 241 includes a rotating shaft 243. The insertion portion 222 is provided with a shaft hole 224. The rotating shaft 243 is mounted in the shaft hole 224. The rotating shaft 243 rotates in the shaft hole 224 to cause the clipping portion 240 to rotate, so that the separation member 200 is finally detachably connected to the surgical instrument 100. The rotation axis is a central axis of the rotating shaft 243.

The shaft hole 224 is partially open, and the rotating shaft 243 is partially accommodated in the shaft hole 224. That is to say, a circumferential extension angle of the shaft hole 224 is less than 360 degrees. For example, the circumferential extension angle of the shaft hole 224 is 180 degrees. The shaft hole 224 is partially open, so that an operation of mounting the rotating shaft 243 into the shaft hole 224 is very convenient, and a size of the separation member 200 may also be reduced, to cause a structure of the separation member 200 to be more compact.

The first end 241 of the clipping portion 240 is mounted to a first side surface 229 of the insertion portion 222. It is to be noted that, the first side surface 229 refers to a surface of a back side of the insertion portion 222, and the surface is not only limited to a plane area indicated by 229. The second end 242 of the clipping portion 240 protrudes from a second side surface 227 of the insertion portion 222. It is to be noted that, the second side surface 227 refers to a surface of a front side of the insertion portion 222, and the surface is not only limited to a plane area indicated by 227. The insertion portion 222 is provided with an opening 228 penetrating the first side surface 229 and the second side surface 227. The second end 242 protrudes from the second side surface 227 by extending through the opening 228 from the first side surface 229.

Therefore, the opening 228 provides space for movement stroke of the second end 242 of the clipping portion 240. That is to say, a thickness of the insertion portion 222 provides space for the movement stroke of the second end 242 of the clipping portion 240. In this way, the structure is reliable; and the size of the separation member 200 may also be reduced, so that the design of the structure is very rational.

The second end 242 of the clipping portion 240 is in elastically-mated connection with the clipping port 60 of the surgical instrument 100. In this implementation, the clipping portion 240 itself has no elasticity. The separation member 200 further includes a support portion 250 abutting against the clipping portion 240. The support portion 250 elastically supports the clipping portion 240. The support portion 250 at least partially made of an elastic material. The support portion 250 provides elastic force for the clipping portion 240. That is to say, the clipping portion 240 only needs to realize a function of being engaged with or detached from the surgical instrument 100. The support portion 250 only needs to realize a function of providing the elastic force. The clipping portion 240 and the support portion 250 perform their own functions, so that process requirements for the clipping portion 240 and the support portion 250 can be reduced, which is more conform to mass production demand.

In order to apply acting force toward the second side surface 227 to the clipping portion 240, to cause the clipping portion 240 to be held in the clipping port 60 without external force, so as to be maintained in the first state of being connected to the surgical instrument 100, the support portion 250 is disposed on the first side surface 229 of the clipping portion 240 and is away from the second side surface 227 relative to the clipping portion 240.

Specifically, the support portion 250 has a first portion 251 and a second portion 252 that are connected to each other. The first portion 251 is connected to the first side surface 229. The second portion 252 abuts against the clipping portion 240, and provides the elastic force for the clipping portion 240, so as to cause the clipping portion 240 to have a trend to be clipped into the clipping port 60.

In order to cause the support portion 250 to be reliably fixed on the separation member 200, a connection portion 260 is disposed to the mounting portion 220. The support portion 250 and the mounting portion 220 are fixed through welding at the connection portion 260. Those skilled in the art may think that, other proper fixing method may also be used according to materials of the support portion 250 and the mounting portion 220.

As described above, the shaft hole 224 used for mounting the rotating shaft 243 is partially open. In order to prevent the rotating shaft 243 from falling off the open shaft hole 224, the support portion 250 covers the open portion of the shaft hole 224. Specifically, the connection portion 260 is disposed adjacent to the shaft hole 224. The connection portion 260 is away from the second end 242 of the clipping portion 240 relative to the shaft hole 224. The far end of the first portion 251 of the support portion 250 is fixed on the connection portion 260. Therefore, the support portion 250 extending from the far end to the near end may naturally cover the shaft hole 224, so that the design of the structure is very rational.

It may be seen that, a position of one portion of the rotating shaft 243 is defined by the shaft hole 224, and a position of the other portion is defined by the support portion 250. Compared with the support portion 250 superposed on the totally-closed shaft hole 224, the size of the separation member 200 can be obviously reduced, so that the structure of the separation member 200 can be more compact.

It is to be noted that, the present invention focuses on reducing the size of the separation member 200 in a plurality of aspects. Although the reduction in size of these designs may not be noticeable to naked eyes (for example, less than 1 mm), the size of the separation member 200 is critical. The reason is that: the end effector 18 of the surgical instrument 100 and the separation member 200 are required to enter the body of the patient by extending through the cannula of the puncture outfit for operation; in order to achieve the purpose of minimally invasive surgery, the cannula of the puncture outfit is available in standard sizes (for example, 5 mm/10 mm/12 mm/15 mm); and widths of the end effector 18 and the separation member 200 must be less than a diameter of the cannula of the puncture outfit, so that when designing the end effector 18 and the separation member 200, a designer needs to repeatedly consider and optimize the structure to achieve the purpose even if the size is reduced by less than 1 mm.

As described above, the first end 241 of the clipping portion 240 is mounted to the first side surface 229 of the insertion portion 222. The first portion 251 of the support portion 250 is connected to the first side surface 229. In order to better arrange the clipping portion 240 and the support portion 250, the first side surface 229 of the insertion portion 222 is recessed inward to form accommodating space 226. That is to say, the accommodating space 226 is disposed at the insertion portion 222 of the mounting portion 220, and partial clipping portion 240 is accommodated in the accommodating space 226. The accommodating space 226 includes the opening 228 penetrating the insertion portion 222. The second end 242 of the clipping portion 240 protrudes from the opening 228 to be in clipped connection with the clipping port 60 in the first state. When the separation member 200 is in the first state, partial clipping portion 240 is accommodated in the accommodating space 226. Preferably, when the separation member 200 is in the first state, partial support portion 250 is also accommodated in the accommodating space 226.

Since the accommodating space 226 may store at least partial clipping portion 240, or even store partial support portion 250, the clipping portion 240 and the support portion 250 are prevented from completely protruding from the first side surface 229 of the insertion portion 222. By rationally using the space of the insertion portion 222 itself, not only the size of the insertion portion 222 can be reduced, but also the structure of the insertion portion 222 can be more regular. The accommodating space 226 accommodates partial clipping portion 240, and may also achieve limitation to the clipping portion 240. The second end 242 of the clipping portion 240 is located in the opening 228, so that the second end 242 may change the position under the action of the elastic force to achieve its function. Preferably, partial support portion 250 is accommodated in the accommodating space 226; and the support portion may be prevented from interfering with the insertion channel when the insertion portion is inserted into the insertion channel.

In this implementation, the insertion portion 222 and the clipping portion 240 have no elasticity. The separation member 200 further includes the elastomer abutting against the clipping portion 240. The elastomer abuts against the support portion 240 to elastically support the clipping portion 240. The elastomer is the support portion 250. The support portion 250 at least partially made of an elastic material. The support portion 250 provides elastic force for the clipping portion 240. Those skilled in the art may think that, the clipping portion 240 may also have the elasticity, and any solution that is the same as or similar to this implementation is covered within the protection scope of the present invention.

In this implementation, there is no other elastomer between the support portion 250 and the clipping portion 240, and the elastic force is provided for the clipping portion 240 only by the support portion 250. That is to say, the elastomer abutting against the clipping portion 240 and elastically supporting the clipping portion 240 only includes the support portion 250. Those skilled in the art may think that, on the basis of disposing the support portion 250, another elastomer is disposed between the support portion 250 and the clipping portion 240, for example, second elastomer. The second elastomer and the support portion 250 provide the elastic force for the clipping portion 240 together. Alternatively, the support portion 250 is not disposed, and the elasticity may also be provided for the clipping portion 240 only by other elastomer. Any solution that is the same as or similar to this implementation is covered within the protection scope of the present invention.

The second end 242 of the clipping portion 240 includes a clipping surface 244 and a slope 246 disposed with the clipping surface 244 at an angle. As shown in Fig. 3, the clipping surface 244 extends vertically. As shown in Fig. 2, Fig. 3 and Fig. 6, the clipping surface 244 abuts against the sidewall of the clipping port 60 to prevent the separation member 200 from falling off the surgical instrument 100. As shown in Fig. 3, the slope 246 obliquely extends upward in a direction from the near end to the far end. The upward here refers to a direction toward the opening of the clipping port 60. Under the guide of the slope 246, the position of the second end 242 is changed due to the rotation of the clipping portion 240 in a process of being inserted into the insertion channel 50, so that the insertion portion 222 of the separation member 200 and the clipping portion 240 may be inserted into the insertion channel 50 more smoothly in the direction from the far end to the near end.

The second end 242 of the clipping portion 240 further includes a top surface 248 disposed between the clipping surface 244 and the slope 246. The top surface 248 has a certain width, and may cause the structure of the second end 242 to be more reliable. The top surface 248 may be a straight surface, or may be the inclined slope 246. If the top surface 248 is a slope, an inclined angle of the top surface 248 is preferably less than an inclined angle of the slope 246.

It is to be noted that, in this implementation, the clipping portion 240 is integrally formed. Those skilled in the art may think that, the portions of the clipping portion 240 are independently formed and are connected in a specific connection manner. Any solution that is the same as or similar to this implementation is covered within the protection scope of the present invention.

As described above, the mounting portion 220 of the separation member 200 includes the insertion portion 222 for mounting the clipping portion 240. Referring to Fig. 4 to Fig. 6, the insertion portion 222 extends lengthwise. Correspondingly, the insertion channel 50 extends from the far end surface of the surgical instrument 100 in a direction toward the near end. Specifically, the insertion channel 50 extends from the far end surface of the staple abutting seat 30 in the direction toward the near end.

It is well known to those skilled in the art that, the staple abutting seat 30 is provided with a knife-moving slot 33 extending lengthwise. The knife-moving slot 33 stores partial cutting component of the surgical instrument 100 and guides the movement of the cutting component. The knife-moving slot 33 is disposed in a center position of the staple abutting seat 30 and extends lengthwise. The insertion channel 50, the clipping port 60 and the knife-moving slot 33 communicate with each other and are coaxially disposed, so that a regular structure is achieved.

The clipping port 60 communicates with the near end of the insertion channel 50 and penetrates the sidewall of the insertion channel 50. Specifically, the clipping port 60 penetrates the sidewall of a staple against surface 31 of the staple abutting seat 30.

Therefore, when the separation member 200 is required to be mounted to the surgical instrument 100, the insertion portion 222 of the separation member 200 is aligned to the insertion channel 50, and the separation member 200 is pushed in the direction from the far end to the near end. During pushing, the insertion channel 50 abuts against the slope 246 of the second end 242 of the clipping portion 240, so that the clipping portion 240 rotates by overcoming the elastic force provided by the support portion 250, until the second end 242 of the clipping portion 240 and the insertion portion 222 are successfully inserted into the insertion channel 50. The separation member 200 is continuously pushed, until the second end 242 of the clipping portion 240 is flush with the clipping port 60. The second end 242 of the clipping portion 240 rotates in a reverse direction under the action of the elastic force provided by the support portion 250, and the second end 242 protrudes from the second side surface 227 of the insertion portion 222 and is clipped in the clipping port 60. In this way, the separation member 200 is mounted in place.

The sidewall of the insertion channel 50, specifically, the sidewall of the insertion channel 50 located on the staple against surface 31 of the staple abutting seat 30 includes a first excavated portion 51 and a second excavated portion 52 that communicate with the clipping port 60. The second excavated portion 52 is located on the far end relative to the first excavated portion 51. A width of the second excavated portion 52 is greater than a width of the first excavated portion 51. A width of the clipping port 60 is greater than the width of the first excavated portion 51.

During the inserting of the separation member 200, the wider second excavated portion 52 may cause the insertion operation of the separation member 200 to be easier. During the moving of the separation member 200 in the second excavated portion 52, the clipping portion 240 is not required to overcome the elasticity of the support portion 250 to rotate, and the support portion 250 is not required to be deformed, so that the service life of the separation member 200 may be prolonged, and the increasing of resistance due to the contact between the clipping portion 240 and the second excavated portion may also be prevented. When the slope 246 of the second end 242 of the separation member 200 is in contact with the first excavated portion 51, the second end 242 of the clipping portion 240 may overcome the elasticity of the support portion 250 to rotate and is moved inside the first excavated portion 51. When the separation member 200 is flush with the clipping port 60 by moving to the second end 242 of the clipping portion 240, since the width of the clipping port 60 is greater than the width of the first excavated portion 51, the clipping surface 244 of the clipping portion 240 may abut against the sidewall of the first excavated portion 51 adjacent to the clipping port 60. In addition, the second end 242 of the clipping portion 240 rotates in the reverse direction under the action of the elastic force of the support portion 250 to protrude from the second side surface 227, so that the second end 242 can be successfully clipped in the clipping port 60, and the separation member 200 is mounted in place.

It may be seen that, through the rational design of the first excavated portion 51 and the second excavated portion 52, not only the service life of the separation member 200 can be prolonged, but also the mounting operation of the separation member 200 can be easier and more convenient.

A length of the second excavated portion 52 is greater than a length of the first excavated portion 51. That is to say, during the movement of the clipping portion 240 in the second excavated portion 52, the clipping portion 240 is not required to overcome the elasticity of the support portion 250 to rotate, and the support portion 250 is not deformed, so that the service life of the separation member 200 can be prolonged.

The width of the first excavated portion 51 equals to a width of the knife-moving slot 33. The knife-moving slot 33, the clipping port 60, the first excavated portion 51 and the second excavated portion 52 are coaxially disposed, so that a proper position of the separation member 200 after mounting may be guaranteed, the separation member does not deviate from the central axis, and the doctor may conveniently operate the separation member.

The second excavated portion 52, the first excavated portion 51, the clipping port 60, and even the knife-moving slot 33 form a channel groove in the sidewall of the insertion channel 50 extending from the far end to the near end.

A first wedge portion 39 is disposed on the far end of the staple abutting seat 30. The first wedge portion 39 has a first slope 38. A second wedge portion 239 is disposed on the near end of the working portion 230. The second wedge portion 239 has a second slope 238. After the separation member 200 is mounted to the staple abutting seat 30, the first slope 38 abuts against the second slope 238.

Since the first slope 38 abuts against the second slope 238, the first wedge portion 39 is superposed with the second wedge portion 239, so that the separation member 200 may be mounted on the staple abutting seat 30 more reliably. In addition, the first wedge portion 39 may cause the far end of the staple abutting seat 30 to be thinner relative to other portions. The second wedge portion 239 may cause the near end of the working portion to be thinner relative to other portions. That is to say, even if the first wedge portion 39 is superposed with the second wedge portion 239, a size of the junction is not obviously increased. In an aspect, the superposition causes the mounting to be reliable; and in another aspect, the size is not obviously increased by the superposition, so that the design is very rational.

The second excavated portion 52 is disposed to the first slope 38. The mounting portion 220 extends from the second slope 238 in the direction toward the near end, so that the structure is compact, and the design is rational.

The combined thickness of the first wedge portion 39 and the second wedge portion 239 is equivalent to the thickness of the staple abutting seat 30 adjacent to the near end of the first slope 38, and/or is equivalent to the thickness of the separation member adjacent to the far end of the second slope 238.

Since the thicknesses are equivalent, that is, the thicknesses are approximately equal, a step is prevented from appearing at the junction of the separation member 200 and the staple abutting seat 30, so that the tissue can be prevented from being scratched.

As described above, the separation member 200 includes the mounting portion 220 and the working portion 230. The working portion 230 is used to strip the tissue.

The working portion 230 includes the near end 231 and a far end 232. In a direction from the near end 231 to the far end 232, a width of the working portion 230 gradually decreases. When the working portion 230 strips the tissue, the narrow far end 232 preferentially contacts the tissue, so that the narrow far end 232 may be conveniently inserted into the tissue and strip the target tissue from other tissue, thereby achieving more convenient operation.

At least a portion of the working portion 230 extends in an arc line from the near end 231 to the far end 232. The working portion 230 has a working surface 234 extending in the arc line and recessed inward. By means of the working surface 234 extending in the arc line, the working portion 230 may successfully pass through a gap between the target tissue and other tissue when stripping the tissue, to better guide the end effector 18 to pass through the gap between the target tissue and other tissue, so that the separation member 200 and the surgical instrument 100 can be more convenient in operation.

The working portion 230 extends in the arc line, and the working surface 234 extends in the arc line, so that the back side of a working member also extends in the arc line. The working portion 230 is regular in structure and good-looking in appearance.

Outer contour lines of the working portion 230 and the mounting portion 220 of the separation member 200 have not obvious edges and corners. All of the outer contour lines are rounded, so that the edges and corners may be prevented from cutting the tissue.

As described above, the separation member 200 is mounted on the far end of the surgical instrument 100. The surgical instrument 100 has a mounting end used for mounting the separation member 200. The separation member 200 further includes an elastic member 280. The elastic member 280 abuts against an end surface of the mounting end. Specifically, the elastic member 280 abuts against the end surface of the far end of the staple abutting seat 30.

Since the end surface of the far end of the mounting end is hard and the elastic member 280 is flexible and deformable, when the elastic member 280 abuts against the end surface of the far end of the mounting end, the end surface of the far end of the mounting end may be prevented from scratching the tissue, and the risk that the tissue is sandwiched in a gap between the end surface of the far end of the mounting end and the separation member 200 may also be reduced, so that unnecessary pulling to the tissue can be avoided, thereby preventing the tissue from being damaged.

An outer surface of the elastic member 280 protrudes from an outer surface of the mounting end or is flush with the outer surface of the mounting end. Specifically, the staple abutting seat has the staple against surface 31 and a back surface 32. In this implementation, preferably, the outer surface of the elastic member 280 is flush with the back surface 32 of the staple abutting seat 30 or protrudes from the back surface 32. When the target tissue is stripped from other tissue, since the mounting end on the side of the back surface 32 of the staple abutting seat 30 is easy to scratch other tissue, the outer surface of the elastic member 280 is flush with the back surface 32 of the staple cartridge or protrudes from the back surface 32, so that the end surface of the far end of the mounting end may be prevented from scratching the tissue to the greatest extend, and the risk that the tissue is sandwiched in the gap between the end surface of the far end of the mounting end and the separation member 200 may be reduced to the greatest extend.

The separation member 200 is provided with a slot 290. The mounting end has a first outer surface 91, and the separation member 200 has a second outer surface 202. When the separation member 200 is in the first state, the slot 290 is located at an adjacent portion of the first outer surface 91 and the second outer surface 202. The elastic member 280 is disposed in the slot 290. By using the slot 290, the elastic member 280 may be better mounted without affecting the functions of the elastic member 280.

The outer surface of the elastic member 280 protrudes from the second outer surface 202 or is flush with the second outer surface 202. By using the slot 290, the elastic member 280 may be better mounted, but, there is a risk of scratching the tissue at the near end and/or the far end of the slot 290. By causing the outer surface of the elastic member 280 to protrude the second outer surface 202 or to be flush with the second outer surface 202, the elastic member 280 can be conveniently mounted, and the tissue can also be prevented from being scratched.

A shape of the elastic member 280 matches a shape of the slot 290, so that the tissue can be prevented from being scratched to the greatest extent. The elastic member 280 is in interference fit with the slot 290, so that the elastic member 280 can be mounted reliably, and the elastic member 280 can conveniently protrude from the surface adjacent to the elastic member, thereby preventing the tissue from being scratched to the greatest extent.

The slot 290 extends circumferentially. Therefore, the mounting of the elastic member 280 may be more reliable.

A bottom surface of the slot 290 is rough. Specifically, the bottom surface of the slot includes depressions and/or projections, which may increase the friction force between the slot 290 and the elastic member 280. In this way, the elastic member 280 can be reliably mounted in the slot 290.

A shape of the bottom surface of the slot 290 is symmetrically designed relative to the central symmetry plane in the longitudinal direction of the separation member 200. Since the structure is regular, the elastic member 280 is evenly stressed, so that an effect of preventing tissue scratching can be better achieved.

In this implementation, the elastic member 280 is a component formed independently. The elastic member 280 is manually disposed in the slot 290. Those skilled in the art may think that, the elastic member 280 may also be formed in the slot 290 in a spraying manner. Any solution that is the same as or similar to this implementation is covered within the protection scope of the present invention.

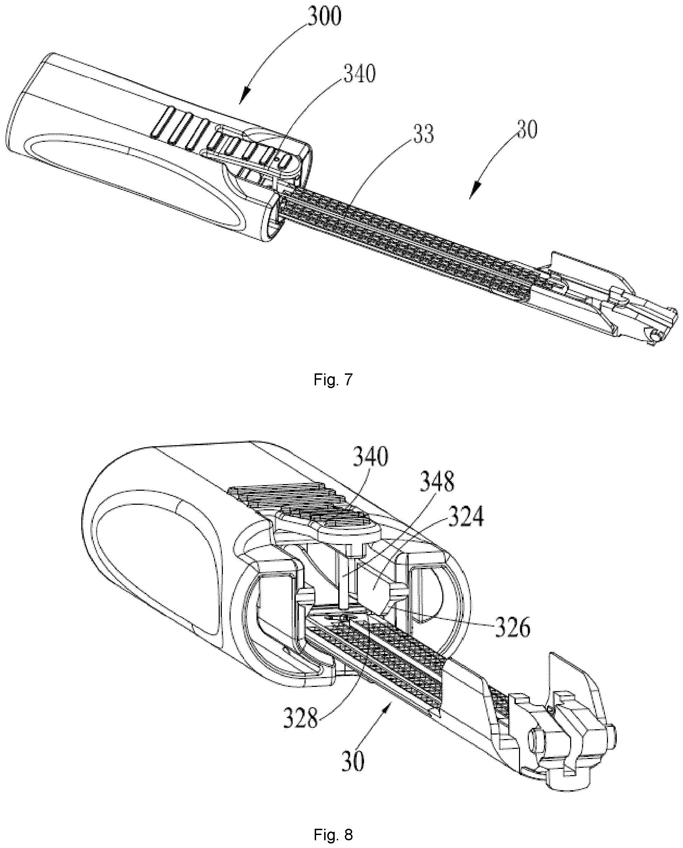

Referring to Fig. 7 to Fig. 13, this implementation further provides a packaging box 300 of the separation member 200. The separation member 200 may be accommodated in the packaging box 300. The packaging box 300 provides an external packing for the separation member 200, so that the separation member 200 is conveniently transported and stored. The separation member 200 and the packaging box 300 form an assembly. The assembly may be sold and purchased separately, or may be sold and purchased together with the surgical instrument 100 to form a surgical kit. Regardless of sale and purchase methods, the needs of the doctor to strip the target tissue can be met. Definitely, the separation member 200 in this implementation may also be sold and purchased separately.

The packaging box 300 includes an accommodation portion 320 used for accommodating the separation member 200. The separation member 200 may be accommodated in the packaging box 300 by inserting the accommodation portion 320. The packaging box 300 includes a housing 350. A surface of a side of the housing 350 is recessed inward to form the open accommodation portion 320. The accommodation portion 320 has an open end, so that the separation member 200 can be conveniently put in or taken out from the packaging box 300 from the open end.

The accommodation portion 320 has a closed end 330 spaced apart from the open end. A shape of the closed end 330 may match a shape of the far end 232 of the separation member 200. The shape of the closed end 330 may also not match the shape of the far end 232 of the separation member 200, as long as the closed end 330 may limit the inserting of the separation member 200. Therefore, during the inserting of the separation member 200 into the accommodation portion 320, when the far end 232 of the separation member 200 abuts against the closed end 330 of the accommodation portion 320, an operator can perceive that the separation member 200 has been accommodated in place.

The accommodation portion 320 is provided with a convex rib 324. The convex rib 324 is provided with a guide surface 326. The separation member 200 slides into the accommodation portion 320 along the guide surface 326. The guide surface 326 may cause the separation member 200 to be inserted into the accommodation portion 320 more easily and accurately.

The convex rib 324 is further provided with an abutment surface 328. The abutment surface 328 and the guide surface 326 intersect with each other and are disposed at an angle, specifically, at an obtuse angle. The abutment surface 328 is close to the closed end 330 of the accommodation portion 320 relative to the guide surface 326. The abutment surface 328 limits the separation member 200. Specifically, when the separation member 200 is mounted in the accommodation portion 320, the abutment surface 328 abuts against partial working surface 234 of the separation member 200, so that the separation member 200 can be accommodated in the accommodation portion 320 more reliably.

It may be seen that, the convex rib 324 may not only define the size of the accommodation portion 320, but also guide the operation of the separation member 200 to slide into the accommodation portion 320. After the separation member 200 slides into the accommodation portion 320, the convex rib 324 may also limit the separation member 200, so that the design of the structure is very rational.

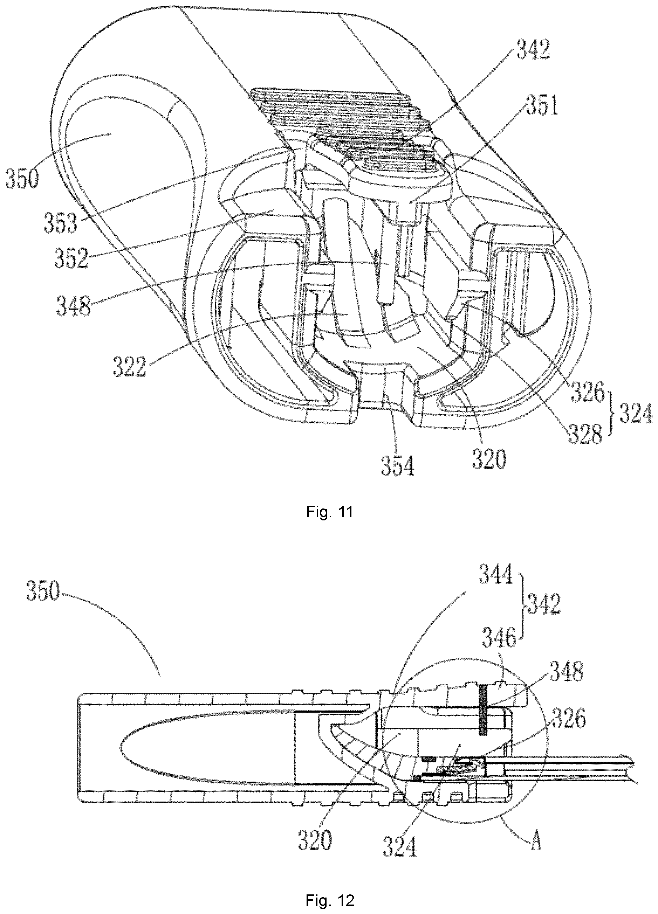

Referring to Fig. 11, a protrusion 322 is disposed on a surface of the accommodation portion 320. The protrusion 322 may support the working portion 230 of the separation member 200, and reduce a contact area between the working portion 230 and the accommodation portion 320, so that the separation member 200 can be inserted or taken out more easily. In this implementation, the protrusion 322 extends lengthwise and is approximately in a strip shape. The protrusion 322 extends in a longitudinal direction. Those skilled in the art may think that, the protrusion 322 may be in other shapes.

As described above, the separation member 200 includes the elastic member 280 abutting against the end surface of the mounting end of the surgical instrument 100. Preferably, the outer surface of the elastic member 280 protrudes from the outer surface of the working portion 230 of the separation member 200. Therefore, the elastic member 280 elastically abuts against an inner surface of the accommodation portion 320.

During the inserting of the separation member 200 into the accommodation portion 320 of the packaging box 300, and when the elastic member 280 elastically abuts against the inner surface of the accommodation portion 320, the operator can perceive the effect of damping. The effect of damping provides operation feedback for the operator, so that the operator may determine whether the separation member 200 is accommodated in place by means of such feedback. That is to say, in addition to the foregoing closed end 330 allowing the operator to perceive that the separation member 200 has been inserted in place, that the elastic member 280 elastically abuts against the inner surface of the accommodation portion 320 further provides feedback for the operator, thereby further improving operation experience. In addition, the friction force between the elastic member 280 and the inner surface of the accommodation portion 320 may prevent the separation member 200 from falling off the accommodation portion 320.

As described above, the separation member 200 is detachably connected to the surgical instrument 100 by using a mating mechanism. The separation member 200 switches between a first state and a second state. In the first state, the separation member 200 is connected to the surgical instrument 100; and in the second state, the separation member 200 is detached from the surgical instrument 100.

In this implementation, preferably, the packaging box 300 may not only accommodate the separation member 200, but also switch the separation member 200 between the first state and the second state. That is to say, the packaging box 300 can not only provide packaging for the separation member 200, but also serve as a disassembling tool of the separation member 100 relative to the surgical instrument 200, of which design is very artful.

Specifically, the packaging box 300 includes an operating member 340. The operating member 340 acts on the mating mechanism to switch the separation member 200 from the first state to the second state. More specifically, the operating portion 342 acts on the clipping mechanism to switch the separation member 200 from the first state to the second state.

The operating member 340 includes the operating portion 342 and a driving portion 348 that are connected to each other. The operating portion 342 is pressed by the operator. The driving portion 348 acts on the clipping mechanism to switch the separation member 200 from the first state to the second state.

The operating portion 342 performs first movement to drive the driving portion 348 to perform second movement, so as to switch the separation member 200 from the first state to the second state. The first movement includes rotation; and the second movement includes rotation.

The packaging box 300 includes the housing 350. The housing 350 is partially recessed inward to form the accommodation portion 320. The housing 350 includes a first sidewall 351 and a second sidewall 352 surrounding the accommodation portion 320. There is a gap 353 between the first sidewall 351 and the second sidewall 352. The first sidewall 351 forms the operating portion 342. The gap 353 extends lengthwise in a direction from one end of the housing 350 to the other end. An extending length of the gap 353 is less than an extending length of the housing 350. Due to the presence of the gap 353, the first sidewall 351 may move relative to the second sidewall 352, so that the driving portion 348 is driven to move and finally acts on the separation member 200 to switch the separation member 200 from the first state to the second state. In addition, the first sidewall 351 is a portion of the housing 350. A portion of the housing 350 forms the operating portion 342, and other components are not required to be extra disposed to form the operating portion 342. Therefore, a simple structure and rational design can be achieved.

The operating portion 342 rotates to drive the driving portion 348 to rotate. Specifically, the first sidewall 351 includes a connection end 344 and a free end 346. The driving portion 348 is connected to the free end 346. The free end 346 rotates around the junction of the connection end 344 and the second sidewall to drive the driving portion 348 to rotate. Extra components are not required to be disposed, so that the simple structure and rational design can be achieved.

A rotation axis of the free end 346 is perpendicular to a central symmetry plane in a longitudinal direction of the packaging box 300. The rotation axis of the free end 346 is also parallel to a rotation axis of the clipping portion 240. Therefore, the assembly formed by the separation member 200 and the packaging box 300 may be regular in structure.

The connection end 344 of the operating portion 342 is made of an elastic material. Optionally, the entire operating portion 342 is made of the elastic material. Therefore, when the free end 346 is pressed, the connection end 344 is deformed, to cause the free end 346 to rotate around the junction of the connection end 344 and the second sidewall. Therefore, the doctor overcomes the elastic force of the operating portion 342 itself to drive the driving portion 348 to move, so that the separation member 200 may be switched from the first state to the second state. By using the elasticity of the operating portion 342 itself, the structure of the packaging box 300 can be simpler. By means of the elasticity of the operating portion 342 itself, the free end 346 is maintained in a raised state without external force, as shown in Fig. 8, Fig. 11 and Fig. 12.

Both the operating portion 342 and the driving portion 348 extend lengthwise. An extending direction of the operating portion 342 is perpendicular to an extending direction of the driving portion 348. The operating portion 342 and the driving portion 348 that extend lengthwise and have the mutually perpendicular extending directions provide an enough stroke, so that the driving portion 348 may effectively the clipping portion 240. Therefore, the separation member 200 can be successfully detached from the surgical instrument 100.

The operating portion 342 is connected to the driving portion 348, and cooperatively abuts against the clipping portion 240. The operating portion 342 and the driving portion 348 are two independent components. Specifically, the driving portion 348 is a pin, and the pin is mated with a hole in the operating portion 342 to achieve the connection between the driving portion 348 and the operating portion 342. Those skilled in the art may think that, the operating portion 342 and the driving portion 348 may also be integrally formed.

As shown in Fig. 11 and Fig. 12, the housing 350 is provided with an opening hole 354 for the driving portion 348 to pass through. The opening hole 354 is disposed opposite to the driving portion 348. Therefore, when the driving portion 348 is mounted, the driving portion 348 is connected to the operating portion 342 after extending through the opening hole 354. Through the opening hole 354, a mounting tool can conveniently extend to apply force to the driving portion 348, so that the structure is rational in design. The driving portion 348 is connected to the free end 346 of the operating portion 342. Correspondingly, the opening hole 354 is opposite to the free end 346 of the operating portion 342. Therefore, rational layout can be achieved.

A width of the driving portion 348 is less than a width of the channel groove (including the second excavated portion 52, the first excavated portion 51 and the clipping port 60 that successively communicate with each other) in the staple abutting seat 30. During the detaching of the separation member 200 from the surgical instrument 100, the driving portion 348 may be moved in the channel groove and maintained in a state of pressing the second end 242 of the clipping portion 240.

It may be learned that, the operating member 340 acts on the clipping mechanism to switch the separation member 200 from the first state to the second state.

The far end of the accommodation portion 320 is closed to form the closed end 330. When the separation member 200 is mounted to the surgical instrument 100, the packaging box 300 accommodating the separation member 200 drives the separation member 200 to move toward the surgical instrument 100. The closed end 330 pushes the separation member 200 to insert into the surgical instrument 100. Therefore, the closed end 330 of the accommodation portion 320 of the packaging box 300 pushes the separation member 200 to switch from the second state to the first state.

The packaging box 300 is made of the transparent material, so that the packaging box is good-looking in appearance. When the separation member 200 is dismounted by using the packaging box 300, the transparent packaging box 300 facilitates the operator to observe whether the dismounting operation is in place.

An application scenario of the separation member 200 in this implementation is described below.

After the separation member 200 leaves factory and before the separation member is used, the separation member 200 is located in the accommodation portion 320 of the packaging box 300. The elastic member 280 on the separation member 200 elastically abuts against the inner side surface of the accommodation portion 320. The separation member 200 is held in the packaging box 300 under the action of the elastic force of the elastic member 280.

When the doctor needs to use the separation member 200 to strip the tissue during the using of the surgical instrument 100, the doctor holds the housing 350 of the packaging box 300, to align the mounting portion 220 of the separation member 200 with the insertion channel 50 disposed on the staple abutting seat 30 of the end effector 18 of the surgical instrument 100, and pushes the packaging box 300 in a direction toward the surgical instrument 100. During pushing, the insertion portion 222 of the separation member 200 and the clipping portion 240 are moved along the insertion channel 50, until the second end 242 of the clipping portion 240 is flush with the clipping port 60 and clipped in the clipping port 60. In this case, the separation member 200 has been mounted on the surgical instrument 100.

Then, the doctor overcomes the friction force between the elastic member and the inner side surface of the accommodation portion 320 due to the elastic force of the elastic member 280, to pull the packaging box 300 in a direction away from the surgical instrument 100, so as to detach the packaging box 300 from the separation member 200, so that the doctor may use the separation member 200 to strip the target tissue. Specifically, the narrow far end 232 of the working portion 230 of the separation member 200 is aligned with an interface of the target tissue and other tissue, and the surgical instrument 100 and the separation member 200 are pushed forward, until the separation member 200 strips the target tissue from other tissue.

After the separation member 200 strips the target tissue from other tissue, the doctor moves forward the surgical instrument 100 in a direction toward the far end 232, to cause the target tissue to be between the staple abutting seat 30 and the staple cartridge base. Then, the doctor may use the surgical instrument 100 to cut and suture the target tissue.

When the doctor does not need to strip the tissue during the using of the surgical instrument 100, the separation member 200 may be taken down from the surgical instrument 100, to prevent the separation member 200 from occupying space to affect other operations when the surgical instrument 100 does not need the separation member 200. Specifically, the doctor aligns the packaging box 300 with the separation member 200 to move to accommodate the separation member 200 in the accommodation portion 320 of the packaging box 300. Then, the doctor presses the free end 346 of the operating portion 342, and then the free end 346 rotates around the connection end 344 to drive the driving portion 348 to rotate, until the driving portion 348 abuts against the slop 246 or the top surface 248 of the second end 242 of the clipping portion 240. The free end 346 of the operating portion 342 is continuously pressed, and the driving portion 348 drives the second end 242 of the clipping portion 240 to rotate against the acting force of the support portion 250, until the second end 242 of the clipping portion 240 is detached from the clipping port 60. Then, the doctor pulls the packaging box 300 in the direction away from the surgical instrument 100. The packaging box 300 drives the insertion portion 222 of the separation member 200 to be detached from the insertion channel 50, so that the separation member 200 is detached from the surgical instrument 100. The separation member 200 detached from the surgical instrument 100 is located in the accommodation portion 320 of the packaging box 300, which returns back to a state before the separation member is used and after the separation member leaves factory. It is to be noted that, after the driving portion 348 causes the second end 242 of the clipping portion 240 to be detached from the clipping port, the driving portion 348 maintains the state of pressing the second end 242 of the clipping portion 240. That is to say, during the detaching of the separation member 200 from the surgical instrument 100, the driving portion 348 presses the second end 242 of the clipping portion 240 all the time and applies force to the second end 242. The driving portion 348 moves in the channel groove. Finally, the packaging box 300 drives the separation member 200 to detach from the surgical instrument 100.

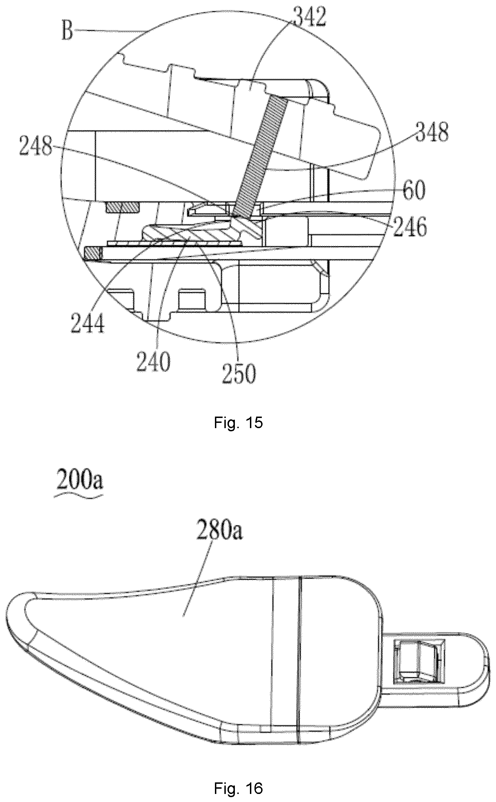

Fig. 16 to Fig. 17 show the separation member 200a cooperatively used with the surgical instrument provided in a second implementation of the present invention.

A difference between the separation member 200a of this implementation and the separation member in the first implementation is mainly described below.

In the first implementation, the elastic member circumferentially extends into an annular shape. The annular elastic member is mounted in a slot between the separation member and the far end surface of the staple abutting seat.

In this implementation, the elastic member 280a is in a bag shape. At least a portion of the working portion 230a is inserted in the elastic member 280a. The at least a portion of the working portion 230a has a same shape as the elastic member 280a. Specifically, the working portion 230a is inserted in the elastic member 280a from the far end to a portion adjacent to the mounting end of the surgical instrument. The end surface of the opening on the near end of the elastic member 280a abuts against the far end surface of the mounting end, to prevent the far end surface of the mounting end from scratching the tissue.

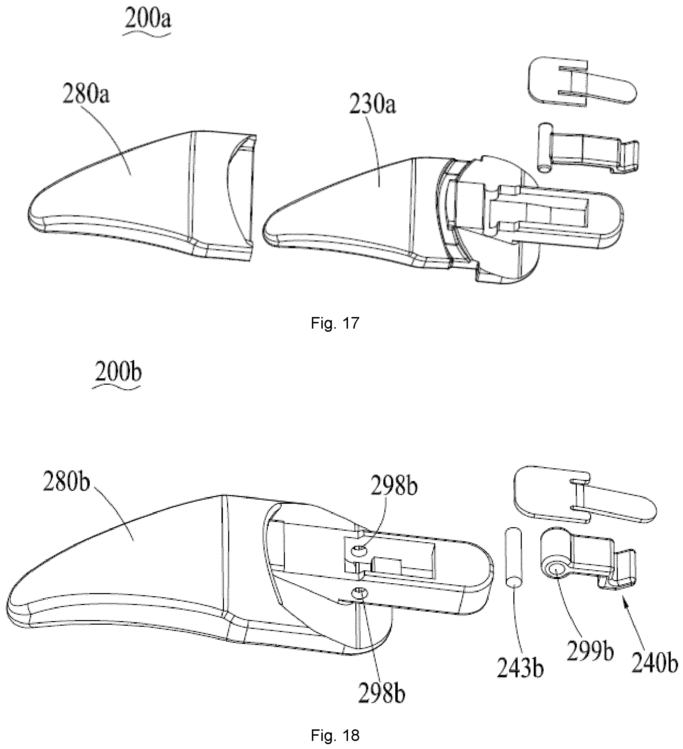

Fig. 18 shows the separation member cooperatively used with the surgical instrument provided in a third implementation of the present invention.

A difference between the separation member 200b of this implementation and the separation member in the first implementation is mainly described below.

In the first implementation, a first end of the clipping portion includes a rotating shaft. The rotating shaft and other portions of the clipping portion are integrally formed. The insertion portion is provided with an open shaft hole. The rotating shaft is partially accommodated in the shaft hole. The support portion covers the open portion of the shaft hole, so that a second end of the clipping portion can rotate around the rotating shaft.

In this implementation, the separation member 200b includes an independent rotating shaft 234b. The first end of the clipping portion 240b is provided with a pin hole 299b extending through the rotating shaft 234b. The mounting portion of the separation member 200b is provided with circular holes 298b corresponding to two end portions of the pin hole 299b. The rotating shaft 234b successively extends through the first circular hole 298b, the pin hole 299b and the second circular hole 298b, so that the first end of the clipping portion 240b is rotatably mounted on the mounting portion. The rotating shaft 234b is mated with the first circular hole 298b and/or second circular hole 298b, to prevent the rotating shaft from falling off.

In addition, in this implementation, the shape of the elastic member 280b of the separation member 200b, the elastic member 280b and the mating mechanism of the working portion 230b are the same as that in the second implementation, which are not described again.

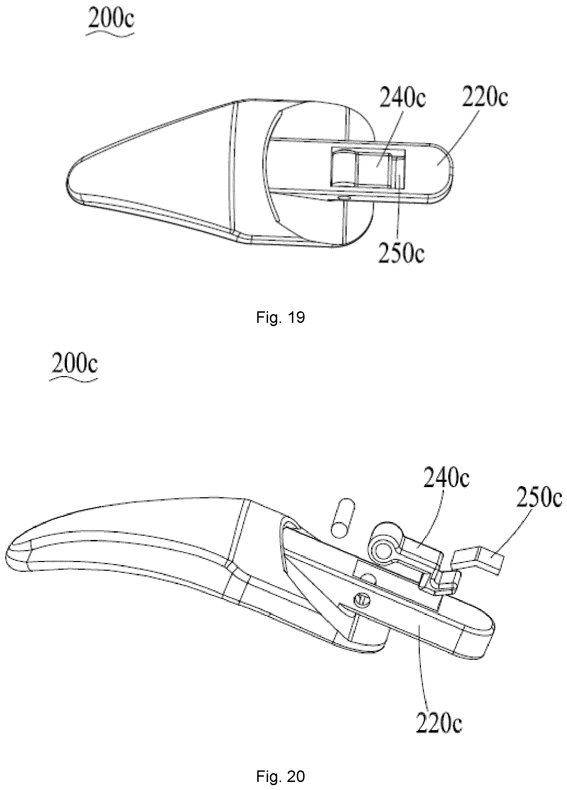

Fig. 19 to Fig. 20 show the separation member cooperatively used with the surgical instrument provided in a fourth implementation of the present invention.

A difference between the separation member 200c of this implementation and the separation member in the third implementation is mainly described below.

In the third implementation, the connection portion is disposed on a far side of the mounting portion of the separation member adjacent to the first end of the clipping portion. The first portion of the support portion is connected to the connection portion. The support portion extends toward the near end from the connection portion. The support portion applies the force to the clipping portion relative to the second portion of the near end, so that the clipping portion has a trend to be clipped in the clipping port of the surgical instrument.

In this implementation, the support portion 250c is disposed close to the near end of the clipping portion 240c. The near end of the support portion 250c is connected to the mounting portion 220c in a manner of welding. The support portion 250c has elasticity. The far end of the support portion 250c applies the force to the second end of the clipping portion 240c, so that the clipping portion 240c has a trend to be clipped in the clipping port 60c in the surgical instrument.

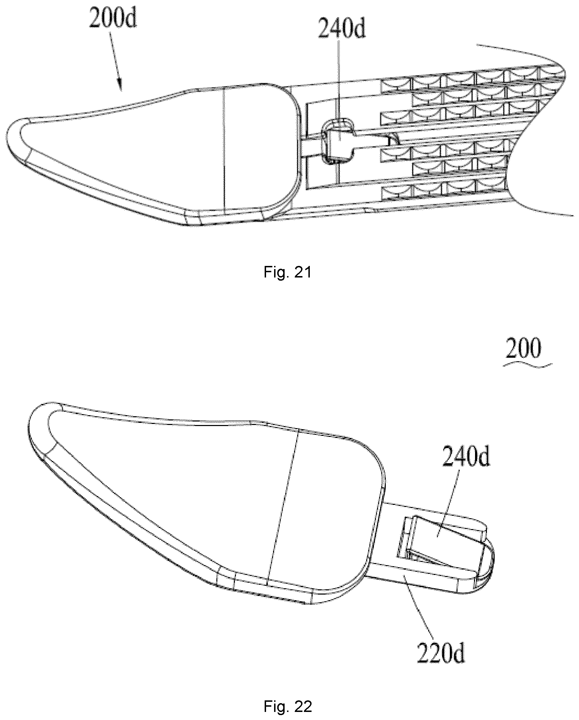

Fig. 21 to Fig. 22 show the separation member cooperatively used with the surgical instrument provided in a fifth implementation of the present invention.

A difference between the separation member 200d of this implementation and the separation member in the second implementation is mainly described below.

In the second implementation, the clipping portion itself has no elasticity, so that the support portion provides the elastic force for the clipping portion to cause the clipping portion to have a trend to be clipped in the clipping port of the surgical instrument.

In this implementation, the clipping portion 240d is at least partially made of the elastic material, so that the clipping portion 240d has a trend to be clipped in the clipping port of the surgical instrument. That is to say, other elastomers are not extra disposed to provide the elastic force for the clipping portion 240d, so that the structure is simpler.

In this implementation, the near end of the clipping portion 240d is connected to the mounting portion 220d of the separation member 200d. The clipping portion 240d is deformed. The far end of the clipping portion 240d rotates around the near end, so that the clipping portion 240d is clipped in the clipping port or detached from the clipping port, and finally, the separation member 200d is switched between the first state and the second state. It is to be noted that, extra rotating shaft is not disposed on the near end, and the far end rotates around the junction of the near end and the mounting portion 220d.

Therefore, it may be seen that, the separation member 200d in this implementation is simpler in structure and lower in cost.

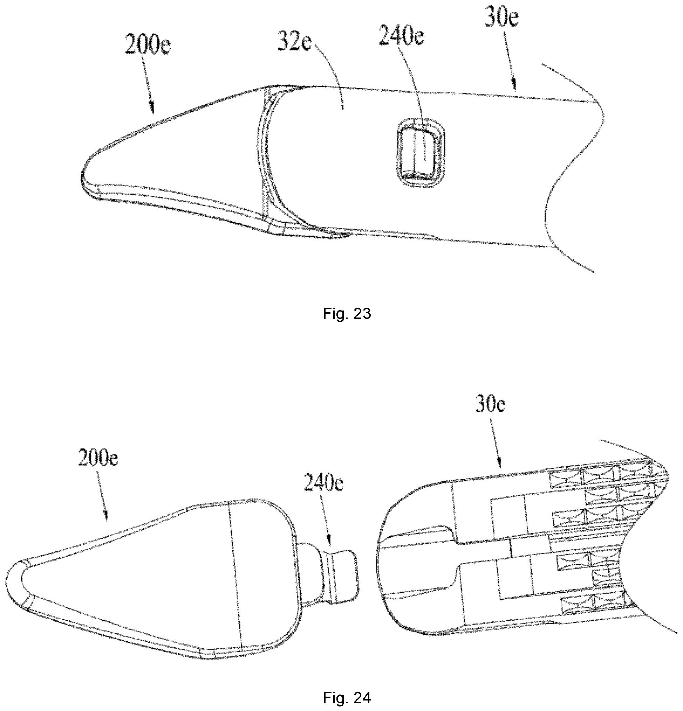

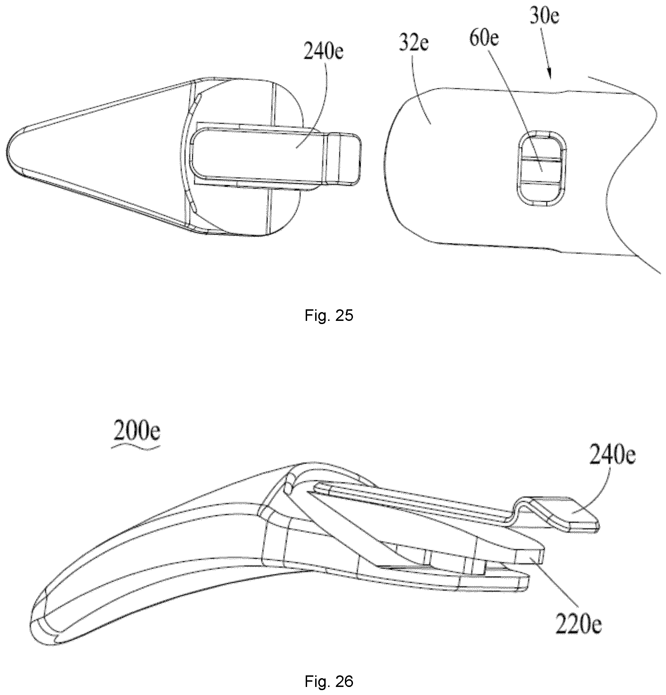

Fig. 23 to Fig. 26 show the separation member cooperatively used with the surgical instrument provided in a sixth implementation of the present invention.

A difference between the separation member 200e of this implementation and the separation member in the fifth implementation is mainly described below.

In the fifth implementation, the clipping portion is disposed on the front surface (second side surface) of the mounting portion of the separation member, and is mated with the clipping port disposed in the surgical instrument.

In this implementation, the clipping portion 240e is disposed on the back surface of the mounting portion 220e of the separation member 200e. The clipping port 60e is disposed on the back surface 32e of the staple abutting seat 30e. The clipping portion 240e disposed on the back surface of the mounting portion 220e is mated with the clipping port 60e disposed on the back surface 32e of the staple abutting seat 30e. Therefore, the separation member 200e can be detachably connected to the surgical instrument.

Since the clipping port 60e is disposed on the back surface 32e of the staple abutting seat 30e, the clipping port is not affected by the staple cartridge base. Therefore, the operating member of the packaging box may operate on the back surface 32e of the staple abutting seat 30e, so as to mount and dismount the separation member 200e. Therefore, the operating space is larger, and the operation is more convenient.

The far end of the clipping portion 240e is connected to the separation member 200e in the manner of welding. The clipping portion 240e itself has the elasticity, so that switching between the first state and the second state can be realized, and details are not described.

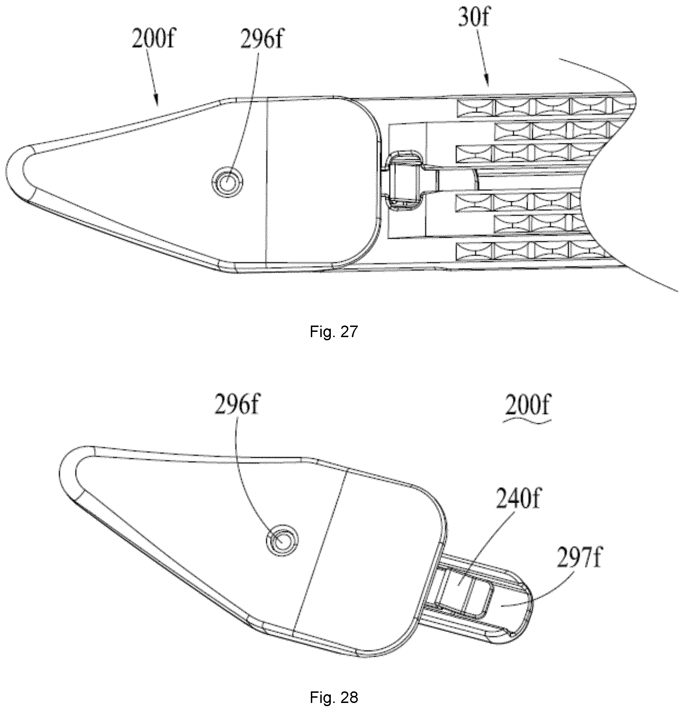

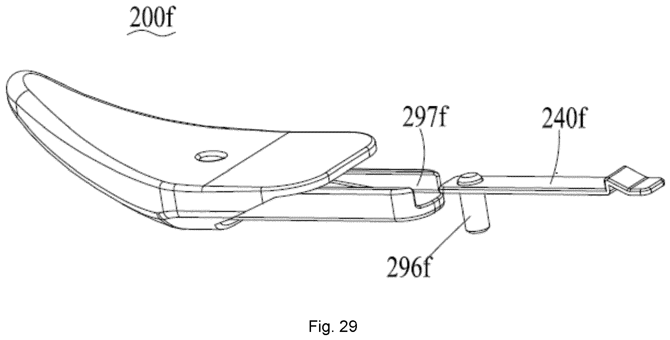

Fig. 27 to Fig. 29 show the separation member cooperatively used with the surgical instrument provided in a seventh implementation of the present invention.

A difference between the separation member 200f of this implementation and the separation member in the fifth implementation is mainly described below.

In the fifth implementation, the near end of the clipping portion is fixed to the mounting portion of the separation member, and the far end of the clipping portion is in mated connection with the clipping port of the surgical instrument.

In this implementation, the far end of the clipping portion 240f is fixed to the portion of the separation member 200f other than the clipping portion 240f, and the near end of the clipping portion 240f is in mated connection with the clipping port of the surgical instrument.

In this implementation, the separation member 200f is provided with an accommodation cavity 297f. The accommodation cavity 297f extends from the near end of the mounting portion in a direction toward the working portion. The accommodation cavity 297f is partially located in the working portion. A mounting shaft 296f vertically extending is disposed on the far end of the accommodation cavity 297f. The clipping portion 240f is provided with a mounting hole that can be sleeved on the mounting shaft 296f. The mounting hole is mated with the mounting shaft 296f to achieve the mated connection between the far end of the clipping portion 240f and the separation member 200f. The near end of the clipping portion 240f may rotate around the far end to achieve the clipping and detaching of the clipping port.

It is to be noted that, in this implementation, although the mounting shaft 296f is a cylindrical shaft and the mounting hole is a circular hole, the clipping portion 240f does not rotate around the axis of the mounting shaft 296f. The rotation axis of the clipping portion 240f is the same as that in the foregoing implementation. The rotation axis of the clipping portion 240f is parallel to a working surface of the separation member 200f.

To sum up, in the present invention, the doctor first uses the separation member to strip the target tissue from other tissue, and then moves the surgical instrument forward, to cause the target tissue to be between the staple cartridge base and the staple abutting seat, so as to operate the surgical instrument to cut and suture the target tissue. By using the separation member to strip the target tissue, the operation of the doctor can be easier and more convenient. In addition, by means of the clipping mechanism, the separation member is in elastically-mated connection with the surgical instrument, so that reliable connection and convenient dismounting can be realized.

In the present invention, the elastomer abuts against the end surface of the mounting end, so that the end surface of the far end of the mounting end may be prevented from scratching the tissue to the greatest extend, and the risk that the tissue is sandwiched in the gap between the end surface of the far end of the mounting end and the separation member may be reduced to the greatest extend.

In the present invention, the packaging box can not only provide packaging for the separation member, but also serve as a disassembling tool of the separation member relative to the surgical instrument, of which design is very artful. In the present invention, the elastomer may not only prevent the tissue from being scratched, but also prevent the separation member from accidentally falling off when the separation member is accommodated in the packaging box.

It should be understood that, although this specification is described in terms of implementations, not every implementation only includes an independent technical solution, and this description in the specification is only for clarity. Those skilled in the art should take the description as a whole, and the technical solutions in each implementation may also be appropriately combined to form other implementations that can be understood by those skilled in the art.

## Claims

1. A surgical kit, comprising an assembly, a surgical instrument (100) and a clipping mechanism, wherein the assembly comprises a separation member (200) and a packaging box (300), the separation member (200) is mounted to the surgical instrument (100) to strip tissue,
wherein the separation member (200) and the surgical instrument (100) switch between a first state and a second state by means of the clipping mechanism; in the first state, the separation member (200) is connected to the surgical instrument (100); in the second state, the separation member (200) is detached from the surgical instrument (100); the packaging box (300) comprises an accommodation portion (320) used for accommodating the separation member (200), and **characterized in that** the packaging box (300) further comprises an operating member (340); and the operating member (340) drives the clipping mechanism to switch the separation member (200) from the first state to the second state.

2. The surgical kit as claimed in claim 1, wherein the operating member (340) comprises an operating portion (342) and a driving portion (348); the operating portion (342) is pressed by an operator; and the driving portion (348) drives the clipping mechanism to switch the separation member (200) from the first state to the second state.

3. The surgical kit as claimed in claim 2, wherein the operating portion (342) performs first movement to drive the driving portion (348) to perform second movement, so as to switch the separation member (200) from the first state to the second state.

4. The surgical kit as claimed in claim 3, wherein the first movement comprises rotation; and the second movement comprises rotation.

5. The surgical kit as claimed in claim 2, wherein the packaging box (300) comprises a housing (350); the accommodation portion (320) is disposed to the housing (350); the housing (350) further comprises a first sidewall (351) and a second sidewall (352) surrounding the accommodation portion (320); there is a gap (353) between the first sidewall (351) and the second sidewall (352); and the first sidewall (351) forms the operating portion (342).

6. The surgical kit as claimed in claim 5, wherein the operating portion (342) comprises a free end (346) and a connection end (344) connected to the second sidewall (352); and the free end (346) rotates around a junction of the connection end (344) and the second sidewall (352), to drive the driving portion (348) to move.

7. The surgical kit as claimed in claim 6, wherein a rotation axis of the free end (346) is perpendicular to a central symmetry plane in a longitudinal direction of the packaging box (300).

8. The surgical kit as claimed in claim 6, wherein at least one portion of the operating portion (342) is made of an elastic material, and the at least one portion of comprises the connection end (344).

9. The surgical kit as claimed in claim 2, wherein an extending direction of the operating portion (342) is perpendicular to an extending direction of the driving portion (348).

10. The surgical kit as claimed in claim 9, wherein the packaging box (300) comprises a housing hole; the housing hole is provided with an opening hole (354); and the opening hole (354) is disposed opposite to the driving portion (348).

11. The surgical kit as claimed in claim 1, wherein the accommodation portion (320) is provided with a guide surface (326); and the guide surface (326) is used to guide the separation member (200) to be inserted into the accommodation portion (320) along the guide surface (326).

12. The surgical kit as claimed in claim 1, wherein the accommodation portion (320) is provided with an abutment surface (328) extending lengthwise; and the abutment surface (328) is used to limit the separation member (200).

13. The surgical kit as claimed in claim 12, wherein the accommodation portion (320) is further provided with a guide surface (326); the guide surface (326) is used to guide the separation member (200) to move into the accommodation portion (320) along the guide surface (326); and the abutment surface (328) and the guide surface (326) intersect with each other and are disposed at an angle.

14. The surgical kit as claimed in claim 1, wherein the accommodation portion (320) has an open end and a closed end (330); the open end is used for inserting the separation member (200) into the accommodation portion (320); and the closed end (330) is used to limit the separation member (200).

15. The surgical kit as claimed in claim 1, wherein a protrusion (322) is disposed on a surface of the accommodation portion (320).

16. The surgical kit as claimed in claim 1, wherein the clipping mechanism comprises a clipping portion (240) disposed to the separation member (200) and a clipping port (60) disposed to the surgical instrument (100); in the first state, the clipping portion (240) is in elastically-mated connection with the clipping port (60); and the operating member (340) drives the clipping portion (240) to detach from the clipping port (60), so that the separation member (200) switches from the first state to the second state.

17. The surgical kit as claimed in claim 1, wherein the packaging box (300) is made of a transparent material.

18. The surgical kit as claimed in any one of claims 1 to 17, wherein the separation member (200) is detachably mounted to the surgical instrument (100) by using a mating mechanism; the mating mechanism comprises an insertion portion (222) disposed to the separation member (200) and an insertion channel (50) disposed to the surgical instrument (100); the mating mechanism further comprises a clipping portion (240) disposed to the separation member (200) and a clipping port (60) disposed to the surgical instrument (100); and when the clipping portion (240) and the insertion portion (222) are inserted into the clipping portion (240) to be in clipped connection with the clipping port (60), the separation member (200) is fixed to the surgical instrument (100).

19. The surgical kit as claimed in claim 18, wherein a sidewall of the insertion channel (50) is provided with a channel groove extending from a far end to a near end; an operating member (340) comprises an operating portion (342) and a driving portion (348); the driving portion (348) drives the clipping portion (240) to detach from the surgical instrument (100); and a width of the driving portion (348) is less than a width of the channel groove.

## Patentansprüche

1. Chirurgisches Kit, umfassend eine Anordnung, ein chirurgisches Instrument (100) und einen Klemmmechanismus, wobei die Anordnung ein Trennungselement (200) und eine Verpackungsschachtel (300) umfasst, wobei das Trennungselement (200) auf dem chirurgischen Instrument (100) montiert ist, um Gewebe abzustreifen,
wobei das Trennungselement (200) und das chirurgische Instrument (100) mittels des Klemmmechanismus zwischen einem ersten Zustand und einem zweiten Zustand umschalten; in dem ersten Zustand das Trennungselement (200) mit dem chirurgischen Instrument (100) verbunden ist; in dem zweiten Zustand das Trennungselement (200) von dem chirurgischen Instrument (100) gelöst ist; die Verpackungsschachtel (300) einen Aufnahmeabschnitt (320) umfasst, der dazu verwendet wird, das Trennungselement (200) aufzunehmen, und **dadurch gekennzeichnet, dass** die Verpackungsschachtel (300) ferner ein Betriebselement (340) umfasst; und das Betriebselement (340) den Klemmmechanismus dazu antreibt, das Trennungselement (200) von dem ersten Zustand in den zweiten Zustand umzuschalten.

2. Chirurgisches Kit nach Anspruch 1, wobei das Betriebselement (340) einen Betriebsabschnitt (342) und einen Antriebsabschnitt (348) umfasst; der Betriebsabschnitt (342) durch einen Betreiber gedrückt wird; und der Antriebsabschnitt (348) den Klemmmechanismus dazu antreibt, das Trennungselement (200) von dem ersten Zustand in den zweiten Zustand umzuschalten.

3. Chirurgisches Kit nach Anspruch 2, wobei der Betriebsabschnitt (342) eine erste Bewegung durchführt, um den Antriebsabschnitt (348) dazu anzutreiben, eine zweite Bewegung durchzuführen, um das Trennungselement (200) von dem ersten Zustand in den zweiten Zustand umzuschalten.

4. Chirurgisches Kit nach Anspruch 3, wobei die erste Bewegung Drehung umfasst; und die zweite Bewegung Drehung umfasst.

5. Chirurgisches Kit nach Anspruch 2, wobei die Verpackungsschachtel (300) ein Gehäuse (350) umfasst; der Aufnahmeabschnitt (320) auf dem Gehäuse (350) platziert ist; das Gehäuse (350) ferner eine erste Seitenwand (351) und eine zweite Seitenwand (352) umfasst, die den Aufnahmeabschnitt (320) umgeben; zwischen der ersten Seitenwand (351) und der zweiten Seitenwand (352) eine Lücke besteht; und die erste Seitenwand (351) den Betriebsabschnitt (342) bildet.

6. Chirurgisches Kit nach Anspruch 5, wobei der Betriebsabschnitt (342) ein freies Ende (346) und ein Verbindungsende (344), das mit der zweiten Seitenwand (352) verbunden ist, umfasst; und sich das freie Ende (346) um eine Verknüpfung des Verbindungsendes (344) mit der zweiten Seitenwand (352) dreht, um den Antriebsabschnitt (348) dazu anzutreiben, sich zu bewegen.

7. Chirurgisches Kit nach Anspruch 6, wobei eine Drehachse des freien Endes (346) senkrecht zu einer zentralen Symmetrieebene in einer Längsrichtung der Verpackungsschachtel (300) ist.

8. Chirurgisches Kit nach Anspruch 6, wobei mindestens ein Abschnitt des Betriebsabschnitts (342) aus einem elastischen Material hergestellt ist und der mindestens eine Abschnitt das Verbindungsende (344) umfasst.

9. Chirurgisches Kit nach Anspruch 2, wobei eine Erstreckungsrichtung des Betriebsabschnitts (342) senkrecht zu einer Erstreckungsrichtung des Antriebsabschnitts (348) ist.

10. Chirurgisches Kit nach Anspruch 9, wobei die Verpackungsschachtel (300) ein Gehäuseloch umfasst; dem Gehäuseloch ein Öffnungsloch (354) bereitgestellt ist; und das Öffnungsloch (354) gegenüber dem Antriebsabschnitt (348) platziert ist.

11. Chirurgisches Kit nach Anspruch 1, wobei dem Aufnahmeabschnitt (320) eine Führungsfläche (326) bereitgestellt ist; und die Führungsfläche (326) dazu verwendet wird, das Trennungselement (200) dazu zu führen, entlang der Führungsfläche (326) in den Aufnahmeabschnitt (320) eingesteckt zu werden.

12. Chirurgisches Kit nach Anspruch 1, wobei dem Aufnahmeabschnitt (320) eine Anlagefläche (328) bereitgestellt ist, die sich in der Länge erstreckt; und die Anlagefläche (328) dazu verwendet wird, das Trennungselement (200) einzuschränken.

13. Chirurgisches Kit nach Anspruch 12, wobei dem Aufnahmeabschnitt (320) ferner eine Führungsfläche (326) bereitgestellt ist; die Führungsfläche (326) dazu verwendet wird, das Trennungselement (200) dazu zu führen, sich entlang der Führungsfläche (326) in den Aufnahmeabschnitt (320) zu bewegen; und sich die Anlagefläche (328) und die Führungsfläche (326) schneiden und angewinkelt platziert sind.

14. Chirurgisches Kit nach Anspruch 1, wobei der Aufnahmeabschnitt (320) ein offenes Ende und ein geschlossenes Ende (330) aufweist; das offene Ende zum Einstecken des Trennungselements (200) in den Aufnahmeabschnitt (320) verwendet wird; und das geschlossene Ende (330) dazu verwendet wird, das Trennungselement (200) einzuschränken.

15. Chirurgisches Kit nach Anspruch 1, wobei ein Vorsprung (322) auf einer Fläche des Aufnahmeabschnitts (320) platziert ist.

16. Chirurgisches Kit nach Anspruch 1, wobei der Klemmmechanismus einen Klemmabschnitt (240), der auf dem Trennungselement (200) platziert ist, und einen Klemmanschluss (60), der auf dem chirurgischen Instrument (100) platziert ist, umfasst; in dem ersten Zustand der Klemmabschnitt (240) in elastisch zusammengefügter Verbindung mit dem Klemmanschluss (60) steht; und das Betriebselement (340) den Klemmabschnitt (240) dazu antreibt, sich von dem Klemmanschluss (60) zu lösen, sodass das Trennungselement (200) von dem ersten Zustand in den zweiten Zustand umschaltet.

17. Chirurgisches Kit nach Anspruch 1, wobei die Verpackungsschachtel (300) aus einem transparenten Material hergestellt ist.

18. Chirurgisches Kit nach einem der Ansprüche 1 bis 17, wobei das Trennungselement (200) unter Verwendung eines Zusammenfügungsmechanismus lösbar auf dem chirurgischen Instrument (100) montiert ist; der Zusammenfügungsmechanismus einen Einsteckabschnitt (222), der auf dem Trennungselement (200) platziert ist, und einen Einsteckkanal (50), der auf dem chirurgischen Instrument (100) platziert ist, umfasst; der Zusammenfügungsmechanismus ferner einen Klemmabschnitt (240), der auf dem Trennungselement (200) platziert ist, und einen Klemmanschluss (60), der auf dem chirurgischen Instrument (100) platziert ist, umfasst; und, wenn der Klemmabschnitt (240) und der Einsteckabschnitt (222) in den Klemmabschnitt (240) eingesteckt werden, um in geklemmter Verbindung mit dem Klemmanschluss (60) zu stehen, das Trennungselement (200) auf dem chirurgischen Instrument (100) befestigt ist.

19. Chirurgisches Kit nach Anspruch 18, wobei einer Seitenwand des Einsteckkanals (50) eine Kanalnut bereitgestellt ist, die sich von einem fernen Ende zu einem nahen Ende erstreckt; ein Betriebselement (340) einen Betriebsabschnitt (342) und einen Antriebsabschnitt (348) umfasst; der Antriebsabschnitt (348) den Klemmabschnitt (240) dazu antreibt, sich von dem chirurgischen Instrument (100) zu lösen; und eine Breite des Antriebsabschnitts (348) geringer als eine Breite der Kanalnut ist.

## Revendications

1. Kit chirurgical, comprenant un ensemble, un instrument chirurgical (100) et un mécanisme de clipsage, dans lequel l'ensemble comprend un élément de séparation (200) et une boîte d'emballage (300), l'élément de séparation (200) étant monté sur l'instrument chirurgical (100) pour dépouiller des tissus,
dans lequel l'élément de séparation (200) et l'instrument chirurgical (100) commutent entre un premier état et un second état au moyen du mécanisme de clipsage ; dans le premier état, l'élément de séparation (200) est relié à l'instrument chirurgical (100) ; dans le second état, l'élément de séparation (200) est détaché de l'instrument chirurgical (100) ; la boîte d'emballage (300) comprend une partie de logement (320) utilisée pour loger l'élément de séparation (200), et **caractérisé en ce que** la boîte d'emballage (300) comprend en outre un élément de fonctionnement (340) ; et l'élément de fonctionnement (340) entraîne le mécanisme de clipsage pour faire passer l'élément de séparation (200) du premier état au second état.

2. Kit chirurgical selon la revendication 1, dans lequel l'élément de fonctionnement (340) comprend une partie de fonctionnement (342) et une partie d'entraînement (348) ; la partie de fonctionnement (342) est pressée par un opérateur ; et la partie d'entraînement (348) entraîne le mécanisme de clipsage pour faire passer l'élément de séparation (200) du premier état au second état.

3. Kit chirurgical selon la revendication 2, dans lequel la partie de fonctionnement (342) effectue un premier mouvement pour entraîner la partie d'entraînement (348) à effectuer un second mouvement, de manière à faire passer l'élément de séparation (200) du premier état au second état.

4. Kit chirurgical selon la revendication 3, dans lequel le premier mouvement comprend une rotation ; et le second mouvement comprend une rotation.

5. Kit chirurgical selon la revendication 2, dans lequel la boîte d'emballage (300) comprend un boîtier (350) ; la partie de logement (320) est disposée sur le boîtier (350) ; le boîtier (350) comprend en outre une première paroi latérale (351) et une seconde paroi latérale (352) entourant la partie de logement (320) ; il y a un espace (353) entre la première paroi latérale (351) et la seconde paroi latérale (352) ; et la première paroi latérale (351) forme la partie de fonctionnement (342).

6. Kit chirurgical selon la revendication 5, dans lequel la partie de fonctionnement (342) comprend une extrémité libre (346) et une extrémité de liaison (344) reliée à la seconde paroi latérale (352) ; et l'extrémité libre (346) tourne autour d'une jonction de l'extrémité de liaison (344) et de la seconde paroi latérale (352), pour entraîner la partie d'entraînement (348) à se déplacer.

7. Kit chirurgical selon la revendication 6, dans lequel un axe de rotation de l'extrémité libre (346) est perpendiculaire à un plan de symétrie central dans un sens longitudinal de la boîte d'emballage (300).

8. Kit chirurgical selon la revendication 6, dans lequel au moins une partie de la partie de fonctionnement (342) est constituée d'un matériau élastique, et l'au moins une partie comprend l'extrémité de liaison (344).

9. Kit chirurgical selon la revendication 2, dans lequel une direction d'extension de la partie de fonctionnement (342) est perpendiculaire à une direction d'extension de la partie d'entraînement (348).

10. Kit chirurgical selon la revendication 9, dans lequel la boîte d'emballage (300) comprend un trou de boîtier ; le trou de boîtier est pourvu d'un trou d'ouverture (354) ; et le trou d'ouverture (354) est disposé à l'opposé de la partie d'entraînement (348).

11. Kit chirurgical selon la revendication 1, dans lequel la partie de logement (320) est pourvu d'une surface de guidage (326) ; et la surface de guidage (326) est utilisée pour guider l'élément de séparation (200) à insérer dans la partie de logement (320) le long de la surface de guidage (326).

12. Kit chirurgical selon la revendication 1, dans lequel la partie de logement (320) est pourvu d'une surface de butée (328) s'étendant dans le sens de la longueur ; et la surface de butée (328) est utilisée pour limiter l'élément de séparation (200).

13. Kit chirurgical selon la revendication 12, dans lequel la partie de logement (320) est en outre pourvue d'une surface de guidage (326) ; la surface de guidage (326) est utilisée pour guider l'élément de séparation (200) afin qu'il se déplace dans la partie de logement (320) le long de la surface de guidage (326) ; et la surface de butée (328) et la surface de guidage (326) se croisent et sont disposées selon un angle.

14. Kit chirurgical selon la revendication 1, dans lequel la partie de logement (320) présente une extrémité ouverte et une extrémité fermée (330) ; l'extrémité ouverte est utilisée pour insérer l'élément de séparation (200) dans la partie de logement (320) ; et l'extrémité fermée (330) est utilisée pour limiter l'élément de séparation (200).

15. Kit chirurgical selon la revendication 1, dans lequel une saillie (322) est disposée sur une surface de la partie de logement (320).

16. Kit chirurgical selon la revendication 1, dans lequel le mécanisme de clipsage comprend une partie de clipsage (240) disposée sur l'élément de séparation (200) et un orifice de clipsage (60) disposé sur l'instrument chirurgical (100) ; dans le premier état, la partie de clipsage (240) est en liaison élastique avec l'orifice de clipsage (60) ; et l'élément de fonctionnement (340) entraîne la partie de clipsage (240) à se détacher de l'orifice de clipsage (60), de sorte que l'élément de séparation (200) passe du premier état au second état.

17. Kit chirurgical selon la revendication 1, dans lequel la boîte d'emballage (300) est constituée d'un matériau transparent.

18. Kit chirurgical selon l'une quelconque des revendications 1 à 17, dans lequel l'élément de séparation (200) est monté de manière amovible sur l'instrument chirurgical (100) en utilisant un mécanisme d'accouplement ; le mécanisme d'accouplement comprend une partie d'insertion (222) disposée sur l'élément de séparation (200) et un canal d'insertion (50) disposé sur l'instrument chirurgical (100) ; le mécanisme d'accouplement comprend en outre une partie de clipsage (240) disposée sur l'élément de séparation (200) et un orifice de clipsage (60) disposé sur l'instrument chirurgical (100) ; et lorsque la partie de clipsage (240) et la partie d'insertion (222) sont insérées dans la partie de clipsage (240) pour être en liaison clipsée avec l'orifice de clipsage (60), l'élément de séparation (200) est fixé à l'instrument chirurgical (100).

19. Kit chirurgical selon la revendication 18, dans lequel une paroi latérale du canal d'insertion (50) est pourvue d'une rainure de canal s'étendant d'une extrémité éloignée à une extrémité proche ; un élément de fonctionnement (340) comprend une partie de fonctionnement (342) et une partie d'entraînement (348) ; la partie d'entraînement (348) entraîne la partie de clipsage (240) à se détacher de l'instrument chirurgical (100) ; et une largeur de la partie d'entraînement (348) est inférieure à une largeur de la rainure de canal.
